# EUROPEAN PATENT APPLICATION

(11) **EP 2 497 475 A1**
(43) Date of publication of application: **12.09.2012**
(21) Application number: 11157551.0
(22) Date of filing: 09.03.2011
(51) Int. Cl.: A61K 31/4365, A61K 31/616, A61P 29/00, A61P 43/00

(54) **Treatment of laminitis with platelet aggregation inhibitors**

(71) Applicant: Dussler, Sabine, 26180 Rastede (DE)
(72) Inventor: Dussler, Sabine, 26180 Rastede (DE)
(74) Representative: Kilger, Ute

(57) **Abstract**

The present invention relates to the novel use of platelet aggregation inhibitors for the treatment and/or prevention of Laminitis in veterinary medicine. In particular the invention refers to the novel use of specific antiplatelet drugs for the treatment and/or prevention of Laminitis.

## Description

The present invention relates to the novel use of platelet aggregation inhibitors for the treatment and/or prevention of Laminitis in veterinary medicine. In particular the invention refers to the novel use of specific antiplatelet drugs for the treatment and/or prevention of Laminitis.

Laminitis is an inflammation of the hoof corium especially with horses but for other ungulates as well and not caused by infectious agents. Since the second half of the 19th century several essays of laminitis appear in monographs, scientific papers and textbooks. In the 19th and 20th century scientific and technical progress promoted the knowledge process of the character and the pathogenesis of laminitis. New possibilities concerning diagnostics and therapy were established. Until today laminitis is especially for horses one of the most important diseases and even with new knowledge and therapies the cure of laminitis is still very problematic.

For bovinae the view concerning the appearance and impact of laminitis changed in the last decades essentially. Nowadays laminitis is regarded as the most important claw disease (Ossent & Lischer, 1998). It works as an underlying disease for many claw disorders. Laminitis - and the local traumatic reasons (Rusterholz 1920) - has been made responsible for the development of sole ulcers (Vermunt 1996). The immune system of e.g. the dairy cow is very vulnerable especially around the time of birth (35 days before calving to 70 days after calving) because of massive changes in its metabolism. Because of diagnostic uncertainties and therefore limited possibilities in practice, basic data for the frequency of the appearance of laminitis are missing.

In other ungulates from families like equidae, bovinae, suidae, deer, ovis and capra laminitis occurs commonly, too.

Concerning the pathogenesis of laminitis the contacts of the corium lamellas become loose from the epidermis lamellas, which physiologically grip into each other like a zipper. Between the coffin bone and the hoof wall appears very high pressure, caused through local edema formation and swelling, caused through higher outlet of tissue liquid and blood cells. Since there is no expansion possibility the high pressure causes heavy pain in that area for the animal.

In this heavy gradient this is called chronic laminitis already after 48 hours.

Further effects of the loosening of the contacts are the sinking and the rotation of the coffin bone in the capsule and a rotation of the coffin joint up until a possible breakthrough of the tip of the coffin bone through the sole with hoof loss, which is a complete detachment of the hoof capsule.

### Causes:

The most common forms of laminitis is laminitis from carbohydrate rich food, but there are as well laminitis after birth, laminitis after poisoning (e.g. through consuming poisonous mushrooms), laminitis of too much exposure to very hard grounds, or laminitis of medicaments (e.g. cortisone). Further there are as well other clinical pictures causing laminitis, e.g. colics, enteritis, lumbago, thyroid diseases or the Cushing's syndrome. Further discussed as causes for laminitis are type of housing of the animals and their psyche (e.g. stress). Further known causes are allergies. On one side, toxins that can be generated through too high protein supply, too much starch or different kinds of sugars in high concentrations (e.g. fructans) can induce laminitis. But on the other side laminitis can as well be induced by consuming of poisonous plants, pesticides, fertilisers or overdoses of medicaments. More frequently are unnatural preserving agents as well as additives of nutrients that are not of the natural habitat of the animals named as factors that can induce laminitis.

Concerning bovinae previous systemic diseases like rumen acidaemia or endometritis are known triggers for laminitis, which cause functional and morphological changes in the capsule.

### Conventional form of therapy:

The therapy of laminitis is mostly pain therapy and promotion of the circulation. Acepromazine and heparin as well as acetylsalicylic acid and phenylbutazone are common medicaments. Diurectics are used in early stages to reduce edemas through dehydration. Another common method is bloodletting of 5 - 10 litres. The goal is the reduction of toxic agents and solid blood components. The taken amount of blood can be replaced by physiological saline or electrolytes. In chronic causes X-ray- and CT-scans give a more detailed view of already developed changes at the hoof. Orthopaedic approaches are plasters, special fittings and special hoof adjustments.

Concerning bovinae the possibilities of therapy are even more reduced. In most of the cases the animals if separated from the herd at all are brought into a box and are supplied analgesic. Eventually bloodletting is conducted. In most cases a possible therapy fails (due to diagnostic methods).

Subject of the present invention is the first successful therapy of laminitis which is the treatment with platelet aggregation inhibitors, preferably in combination with acetylsalicylic acid. As will be shown in the examples below this treatment succeeded in chronic cases as well as in acute attacks of laminitis in all treated horses uniformly- After the treatment the horses were clinically not noticeable. Another factor of the new treatment is that the costs are lower compared to the conventional therapy. The medicament can be given with animal feed for prevention and/or treatment of laminitis.

Platelet aggregation inhibitors are members of a class of pharmaceuticals that decrease platelet aggregation and inhibit thrombus formation. They are effective in the arterial circulation. They are widely used in primary and secondary prevention of thrombotic cerebrovascular or cardiovascular disease.

### New form of therapy:

Treatment with platelet aggregation inhibitors in combination with acetylsalicylic acid succeeded in chronic cases as well as in acute attacks of laminitis in all treated horses uniformly. After the treatment the horses were clinically not noticeable. Another factor of the new treatment is that the costs are lower compared to the conventional therapy. The medicament can be given with animal feed for prevention and/or treatment of laminitis. Further, surprisingly, the medicament can be given to pregnant animals without problems occuring,

Platelet aggregation inhibitors are members of a class of pharmaceuticals that decrease platelet aggregation and inhibit thrombus formation. They are effective in the arterial circulation. They are widely used in primary and secondary prevention of thrombotic cerebrovascular or cardiovascular disease.

In one object of the invention the dosage of treatment can be:
per 60 kg 10 mg Prasugrel and 500 mg acetylsalicylic acid twice a day
   or
per 60 kg 75 mg Clopidogrel and 500 mg acetylsalicylic acid twice a day

An initial dosage of
20 mg Prasugrel and 1000 mg acetylsalicylic acid per 60 kg
or
150 mg Clopidogrel and 1000 mg acetylsalicylic acid per 60 kg
may be given depending of the distinct clinical picture.

One object of the invention is a platelet aggregation inhibitor for use in the treatment and/or prevention of laminitis in ungulates which suffer from an acute attack of laminitis or for the prevention and/or treatment of laminitis in ungulates.

One object of the invention is to treat unglates which suffer from an acute attack of laminitis or for the prevention and or treatment of laminitis.

One embodiment of the invention is a platelet inhibitor or a tautomer thereof, or a pharmaceutically acceptable salt thereof for use in the treatment and/or prevention of laminitis in ungulates.

Another embodiment of the invention is a compound or a tautomer thereof, or a pharmaceutically acceptable salt thereof selected from the group comprising

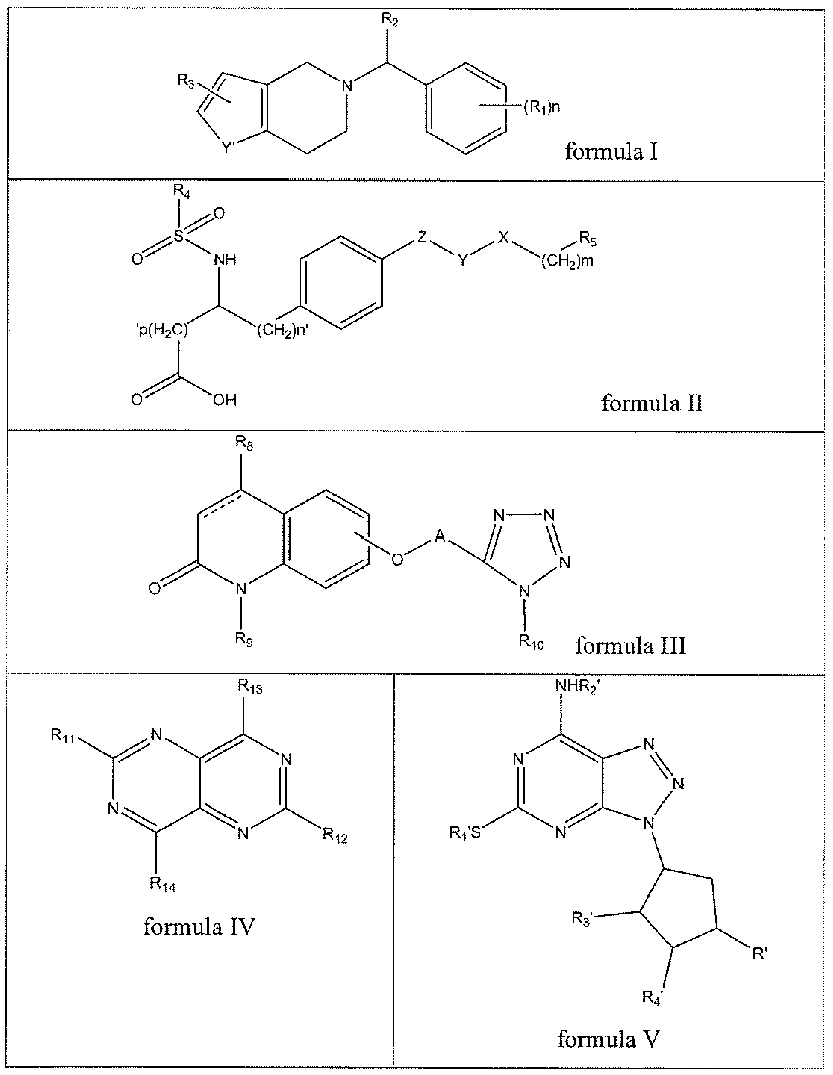

wherein
in formula I
- Y': is NH, O or S;
- R₁: represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl group;
- R₂: represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
- R₃: represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxolen-4yl)methoxy group; a (5-phenyl-2-oxo-1,3-dioxolen-4-yl)methoxy group; a group of formula -NR^{a} R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
- n: is an integer from 1 to 5, and, when n is an integer from 2 to 5, the groups represented by R₁ may be the same as or different from each other;
- said substituents A: are selected from the group consisting of halogen atoms, hydroxy groups, alkoxy groups having from 1 to 4 carbon atoms and cyano groups;
- said substituents B: are selected from the group consisting of alkyl groups having from 1 to 4 carbon atoms, halogen atoms and alkoxy groups having from 1 to 4 carbon atoms;
- said substituents C: are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom;
in formula II
- R₄: is aryl, C₁₋₁₀ alkyl or C₄₋₁₀ aralkyl,
- Z: is a bond or O, -NHC(O)-, -C(O)NH-, C₁₋₅ straight or branched alkyl,
- m: is an integer from 0 to 6
- R₅: is
a) a six membered saturated heterocyclic ring containing one or two heteroatoms wherein said heteroatoms are N or O and wherein said heterocyclic ring is optionally substituted by C₁₋₃ alkyl,
   or
b) NR₆R₇, wherein R₆ and R₇ are independently hydrogen or C₁₋₁₀ alkyl
- X and Y: are independently C₁₋₁₀ alkyl or cycloalkyl, -NHC(O)- or -C(O)NH-
- n': is one or two
- p': is zero or one
in formula III
- R₈: is a hydrogen atom, a lower alkyl group or a group of the formula 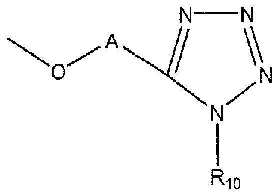
- R₉: is a hydrogen atom, a lower alkyl group, a lower alkenyl group, a lower alkenoyl group, a benzoyl group or a phenyl-C.sub.1-4 alkyl group;
- R₁₀: is a lower alkyl group, a C₃₋₈ cycloalkyl group, a C₃₋₈ cycloalkyl-C₁₋₄ alkyl group, a phenyl group or a phenyl-C₁₋₄ alkyl group;
- A: is a lower alkylene group,
the carbon-carbon bond between 3- and 4-positions in the carbostyril skeleton is either single or double bond; and the substituted position of the group represented by the formula, 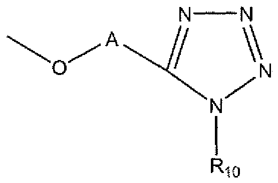 in the carbostyril skeleton is either 4-, 5-, 6-, 7- or 8-position provided that only one such group of the formula can be substituted in the whole carbostyril skeleton, thus when R⁸ in 4-position is 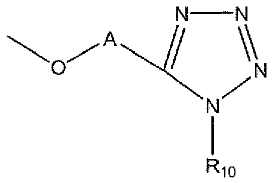 then 5-, 6-, 7- or 8-position will have no such substituted group; furthermore, the phenyl group in the above-mentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy. in formula IV
- R₁₁: is chloro and R₁₂ is diethanolamino, or
- R₁₁/R₁₂: are identical and are selected from allyl, halo, diethanolamino, solketalo and a group having the formula -O-R_{z} or -NHR_{z},
- R_{z}: is selected from alkyl, hydroxyalkyl, alkoxyalkyl, dialkoxyalkyl and 2-oxo-alkyl wherein the or each alkyl and/or alkoxy moiety has less than six carbon atoms, and
- R₁₃/R₁₄: are identical and are selected from piperidino, N-tetrahydroisoquinolyl, and a benzylamino group having the following structural formula: 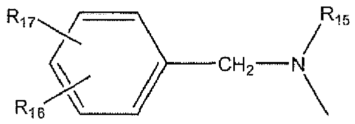 where
R₁₅ is H, or an optionally substituted alkyl or benzyl group, and
R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
- R₁': is a C₁₋₆ alkyl, C₂₋₆ alkenyl, C₂₋₆ alkenyl, C₃₋₈-cycloalkyl or a phenyl group, each group being optionally substituted by one or more substituents selected from halogen, OR₈', NR₉' R₁₀' , SR₁₁'or C₁₋₆ alkyl (itself optionally substituted by one or more halogen atoms);
- R₂': is C₁₋₈ alkyl optionally substituted by one or more substituents selected from halogen, OR₈', NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂, C(O)R₈', OR₈', SR₁₁', NR₁₂'R₁₃', a fused 5- or 6- membered saturated ring containing one or two oxygen atoms, phenyl or C₁₋₆-alkyl the latter two groups being optionally substituted by OR₈', NR₉'R₁₀' or one or more halogen atoms;
- R₃'/R₄': one of them is hydroxy and the other is hydrogen, hydroxy or NR₉'R₁₀' ;
- R': is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅'R₆')ⱼR₁₄' where R₅' and R₆' are as defined above, j is 1 to 3 and R₁₄' is COOH, OR₁₅', NR₁₆'R₁₇' or CONR₁₆'R₁₇'; or R' is a C₁₋₄ alkyl or C₂₋₄ alkenyl group, each or which is substituted by one or more groups selected from =S, =O, =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃' are independently hydrogen, C₁₋₄ alkyl or phenyl; R₂₀' is OR₂₄'or NR₂₅'R_{26'}, where R₂₄' is hydrogen, C₁₋₄ alkyl or phenyl, and R₂₅' and R₂₆" are independently hydrogen, C₁₋₄ alkyl, aryl, C₁₋₆ acyl, arylsulphonyl or arylcarbonyl);
- R₈': is hydrogen, C₁₋₆ alkyl optionally substituted by halogen or R₈' is phenyl optionally substituted by one or more substituents selected from halogen, NO₂, C(O)R₆', OR₆', SR₉', NR¹⁰'R₁₁';
- R₉'/R₁₀'R₁₁': are independently hydrogen or C₁₋₆ alkyl;
- R₁₂'^{/}R₁₃': are independently hydrogen, C₁₋₆ alkyl, acyl, alkyl sulfonyl optionally substituted by halogen, or phenyl sulfonyl optionally substituted by C₁-C₄ alkyl; and
- R₁₅'/R₁₆'/R₁₇': are independently hydrogen C₁₋₆ alkyl;
for the use in the treatment and/or prevention of Laminitis in Ungulates.

One embodiment of the invention is a compound or a tautomer thereof, or a pharmaceutically acceptable salt thereof selected from the group comprising

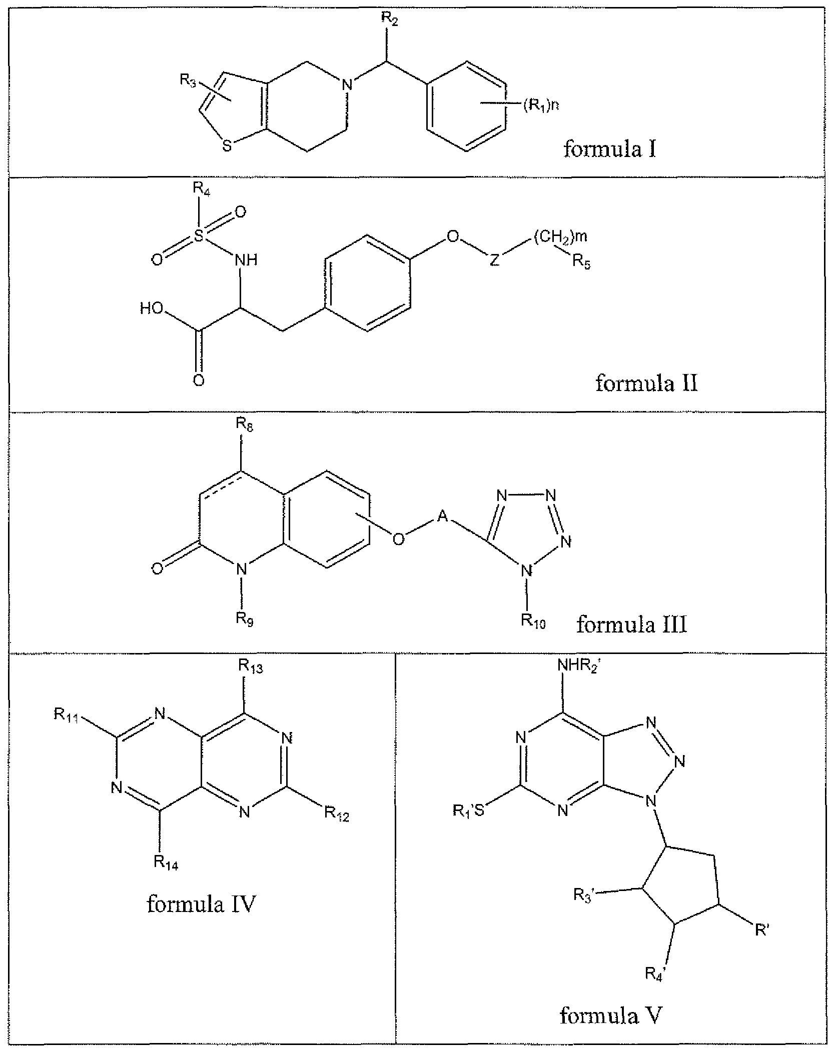

wherein
in formulae I
- R₁: represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl group;
- R₂: represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
- R₃: represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxolen-4yl)methoxy group; a (5-phenyl-2-oxo-1,3-dioxolen-4-yl)methoxy group; a group of formula -NR^{a}R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
- n: is an integer from 1 to 5, and, when n is an integer from 2 to 5, the groups represented by R₁ may be the same as or different from each other;
- said substituents A: are selected from the group consisting of halogen atoms, hydroxy groups, alkoxy groups having from 1 to 4 carbon atoms and cyano groups;
- said substituents B: are selected from the group consisting of alkyl groups having from 1 to 4 carbon atoms, halogen atoms and alkoxy groups having from 1 to 4 carbon atoms;
- said substituents C: are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom;
in formula II
- R₄: is aryl, C₁₋₁₀ alkyl or C₄₋₁₀ aralkyl,
- Z: is a bond or O, -NHC(O)-, -C(O)NH-, C₁₋₅ straight or branched alkyl,
- m: is an integer from 2 to 6
- R₅: is
a) a six membered saturated heterocyclic ring containing one or two heteroatoms wherein said heteroatoms are N or O and wherein said heterocyclic ring is optionally substituted by C₁₋₃ alkyl,
   or
b) NR₆R₇, wherein R₆ and R₇ are independently hydrogen or C₁₋₁₀ alkyl
in formula III
- R₈: is a hydrogen atom, a lower alkyl group or a group of the formula 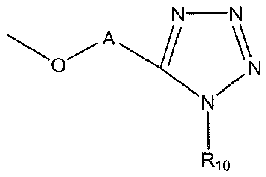
- R₉: is a hydrogen atom, a lower alkyl group, a lower alkenyl group, a lower alkanoyl group, a benzoyl group or a phenyl-C.sub.1-4 alkyl group;
- R₁₀: is a lower alkyl group, a C₃₋₈ cycloalkyl group, a C₃₋₈ cycloalkyl-C₁₋₄ alkyl group, a phenyl group or a phenyl-C₁₋₄ alkyl group;
- A: is a lower alkylene group,
the carbon-carbon bond between 3- and 4-positions in the carbostyril skeleton is either single or double bond; and the substituted position of the group represented by the formula, 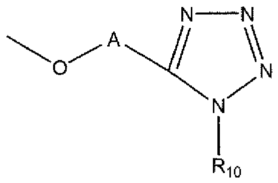 in the carbostyril skeleton is either 4-, 5-, 6-, 7- or 8-position provided that only one such group of the formula can be substituted in the whole carbostyril skeleton, thus when R⁸ in 4-position is 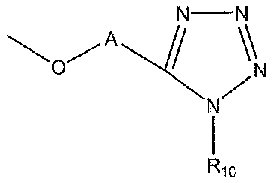 then 5-, 6-, 7- or 8-position will have no such substituted group; furthermore, the phenyl group in the above-znentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy. in formula IV
- R₁₁: is chloro and R₁₂ is diethanolamino, or
- R₁₁/R₁₂: are identical and are selected from allyl, halo, diethanolamino, solketalo and a group having the formula -O-R_{z} or -NHR_{z},
- R_{z}: is selected from alkyl, hydroxyalkyl, alkoxyalkyl, dialkoxyalkyl and 2-oxo-alkyl wherein the or each alkyl and/or alkoxy moiety has less than six carbon atoms, and
- R₁₃/R₁₄: are identical and are selected from piperidino, N-tetrahydroisoquinolyl, and a benzylamino group having the following structural formula: 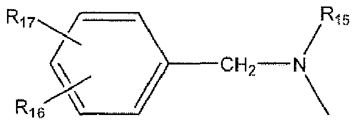 where
R₁₅ is H, or an optionally substituted alkyl or benzyl group, and
R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
- R₁': is a C₁₋₆ alkyl, C₂₋₆ alkenyl, C₂₋₆ alkenyl, C₃₋₈-cycloalkyl or a phenyl group, each group being optionally substituted by one or more substituents selected from halogen, OR₈', NR₉'R₁₀', SR₁₁' or C₁₋₆ alkyl (itself optionally substituted by one or more halogen atoms);
- R₂': is C₁₋₈ alkyl optionally substituted by one or more substituents selected from halogen, OR₈', NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂, C(O)R₈', OR₈', SR₁₁', NR₁₂'R₁₃', a fused 5- or 6- membered saturated ring containing one or two oxygen atoms, phenyl or C₁₋₆-alkyl the latter two groups being optionally substituted by OR₈', NR₉'R₁₀' or one or more halogen atoms;
- R₃'/R₄': one of them is hydroxy and the other is hydrogen, hydroxy or NR₉'R₁₀';
- R': is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅R₆')ⱼR₁₄' where R₅' and R₆' are as defined above, j is 1 to 3 and R₁₄' is COOH, OR₁₅', NR₁₆'R₁₇' or CONR₁₆'R₁₇ '; or R' is a C₁₋₄ alkyl or C₂₋₄ alkenyl group, each or which is substituted by one or more groups selected from =S, =O, =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃ "are independently hydrogen, C₁₋₄ alkyl or phenyl; R₂₀' is OR₂₄' or NR₂₅ 'R₂₆', where R₂₄' is hydrogen, C₁₋₄ alkyl or phenyl, and R₂₅' and R₂₆" are independently hydrogen, C₁₋₄ alkyl, aryl, C₁₋₆ acyl, arylsulphonyl or arylcarbonyl);
- R₈': is hydrogen, C₁₋₆ alkyl optionally substituted by halogen or R₈' is phenyl optionally substituted by one or more substituents selected from halogen, NO₂, C(O)R₆', OR₆', SR₉', NR₁₀'R₁₁';
- R₉'/R₁₀'^{/}R₁₁': are independently hydrogen or C₁₋₆ alkyl;
- R₁₂'^{/}R₁₃': are independently hydrogen, C₁₋₆ alkyl, acyl, alkyl sulfonyl optionally substituted by halogen, or phenyl sulfonyl optionally substituted by C₁-C₄ alkyl; and
- R₁₅'/R₁₆'/R₁₇': are independently hydrogen C₁₋₆ alkyl;

Another embodiment of the invention is a compound or a tautomer thereof, or a pharmaceutically acceptable salt thereof
wherein
in formula I
- R₁: represents a hydrogen atom, an alkyl group having from 1 to 4 carbon atoms, a halogen atom, a fluoroalkyl group having from 1 to 4 carbon atoms and at least one fluorine atom, a hydroxy group, an alkoxy group having from 1 to 4 carbon atoms, a fluoroalkoxy group having from 1 to 4 carbon atoms and at least one fluorine atom, an alkylthio group having from 1 to 4 carbon atoms, a fluoroalkylthio group having from 1 to 4 carbon atoms and at least one fluorine atom, an amino group, an alkanoyl group having from 1 to 5 carbon atoms, a fluoroalkanoyl group having from 2 to 5 carbon atoms and at least one fluorine atom, an alkoxycarbonyl group having from 2 to 5 carbon atoms, a carbamoyl group, a cyano group, a nitro group, an alkanesulfonyl group having from 1 to 4 carbon atoms, a fluoroalkanesulfonyl group having from 1 to 4 carbon atoms and at least one fluorine atom, or a sulfamoyl group
in formula II
- R₄: is aryl, C₁₋₁₀ alkyl or C₄₋₁₀ aralkyl;
- R₅: is
a) a six membered saturated heterocyclic ring containing one or two heteroatoms wherein said heteroatoms are N,
   or
b) NR₆R₇, wherein R₆ and R₇ are independently hydrogen or C₁₋₁₀ alkyl
- Z: is a bond or O, -NHC(O)-, or-C(O)NH-
- m: is an integer from 2 to 6
in formula III
the substituted position of the group of the formula 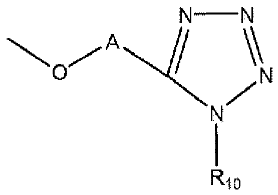 is 5 or 6-position of the carbostyril skeleton
in formula IV
all alkyl groups when present as such, or as a moiety m other groups such as alkoxy in R11-R17 and Rx, Ry and Rz, are lower alkyl groups containing 1-5 carbon atoms
in formula V
- R₁': is C₁₋₄ alkyl or phenyl substituted by trifluoromethyl
for the use in the treatment and/or prevention of Laminitis in Ungulates.

Another embodiment of the invention is a compound or a tautomer thereof, or a pharmaceutically acceptable salt thereof
wherein
in formula I
- R₂: represents hydrogen or an alkanoyl group having from 2 to 6 carbon atoms, a substituted alkanoyl group which has from 2 to 6 carbon atoms and which is substituted by at least one substituent selected from the group consisting of substituents A', defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygen or nitrogene and which may be substituted by an alkanoyl group having from 1 to 10 carbon atoms or a benzyl group, a cycloalkylcarbonyl group having from 4 to 7 carbon atoms, a substituted cycloalkylcarbonyl group which has from 4 to 7 carbon atoms and which is substituted by at least one substituent selected from the group consisting of substituents A', defined below of a substituted benzoyl group having at least one fluorine substituent; and
- said substituents A': are selected from the group consisting of fluorine atoms, chlorine atoms, hydroxy groups, methoxy groups, ethoxy groups and cyano groups
in formula II
- R₄: is C₁₋₁₀ alkyl;
- R₅: is a six membered saturated heterocyclic ring containing one heteroatom wherein said heteroatom is N;
- Z: is a bond or O
- m: is an integer from 2 to 6
in formula III
- R₁₀: is a C₃₋₈ cycloalkyl group or a C₃₋₈ cycloalkyl-C₁₋₄ alkyl group
in formula IV
- R₁₃ and R₁₄: are piperidino, and
- R₁₁ and R₁₂: are each selected from allyl, methoxy, ethoxy, n-propoxy, iso-propoxy, iso-butoxy, 3-methylbutoxy, 2-oxo-n-propoxy, 2,2-diethoxy-n-propoxy, (2-methoxy-1-methyl) ethoxy, 2- methoxyethoxy, 2-hydroxypropoxy, 2-hydroxyethoxy and 3- hydroxypropoxy
in formula V
- R₂': is butyl or cyclopropyl optionally substituted by phenyl, the phenyl group itself being optionally substituted by one or more halogen, C₃₋₈ alkyl, phenoxy or phenyl groups
for the use in the treatment and/or prevention of Laminitis in Ungulates.

Another embodiment of the invention is a compound or a tautomer thereof, or a pharmaceutically acceptable salt thereof
wherein
in formula I
- R₃: represents a hydrogen atom, a hydroxy group, an alkoxy group having from 1 to 4 carbon atoms, an alkoxymethoxy group in which the alkoxy part has from I to 4 carbon atoms, an alkanoyloxymethoxy group in which the alkanoyl part has from 1 to 5 carbon atoms, a benzyloxy group which is unsubstituted or is substituted by at least one substituent selected from the group consisting of substituents D', defined below, an alkanoyloxy group having from 1 to 18 carbon atoms, an alkenoyloxy group having 3 or 4 carbon atoms, a cycloalkylcarbonyloxy group having from 4 to 7 carbon atoms, a benzoyloxy group which is unsubstituted or is substituted by at least one substituent selected from the group consisting of substituents D', defined below, an alkoxycarbonyloxy group having from 2 to 5 carbon atoms, a benzyloxy. carbonyloxy group which is unsubstituted or is substituted by at least one substituent selected from the group consisting of substituents D', defined below, a phthalidyloxy group, a (5-methyl-2-oxo-1,3-dioxalen-4-yl)methoxy group, a (5-phenyl-2-oxo-1,3-dioxolen-4-yl)methoxy group, a group of formula -NR^{a}R^{b}, wherein R^{a} and R^{b} are independently selected from the group consisting of hydrogen atoms, methyl and ethyl groups or R^{a} represents a hydrogen atom and R^{b} represents an alkanoyloxymethyl group in which the alkanoyl part has from 1 to 5 carbon atoms; a benzylamino group, an alkanoylamino group having from 1 to 18 carbon atoms, an alkenoylamino group having 3 or 4 carbon atoms, a cycloalkylcarbonylamino group having 6 or 7 carbon atoms, a benzoylamino group which is unsubstituted or is substituted by at least one substituent selected from the group consisting of substituents D', defined below, an alkoxycarbonylamino group having from 2 to 5 carbon atoms or a benzyloxycarbonylamino group which is unsubstituted or is substituted by at least one substituent selected from the group consisting of substituents D', defined below;
- said substituents D': are selected from the group consisting of fluorine atoms, chlorine atoms, methyl groups and methoxy groups
in formula II
- R₅: is a six membered saturated heterocyclic ring containing one heteroatom wherein said heteroatom is N;
- R₄: is -(CH₂)(CH₂)(CH₂)(CH₃);
- Z: is a bond or O
- m: is 4
in formula III
- R¹⁰: is a phenyl-C₁₋₄ alkyl group which may have substituted group(s) selected from the group consisting of lower alkoxy, lower alkyl, halogen, di-lower alkylamino, nitro, and lower alkenedioxy, a phenyl group which may have substituted group(s) selected from the group consisting of lower alkoxy, lower alkyl, halogen, di-lower alkylamino, nitro, and lower alkenedioxy or a lower alkyl group
in formula IV
- R₁₃ and R₁₄: are N-tetrahydroisoquinolyl and
- R₁₁ and R₁₂: are each selected from diethanolamino and chloro
in formula V
- R₃' and R₄': are both hydroxy
for the use in the treatment and/or prevention of Laminitis in Ungulates.

Another embodiment of the invention is a compound or a tautomer thereof, or a pharmaceutically acceptable salt thereof
wherein
in formula I
- R₁: represents a hydrogen atom, an alkyl group having from I to 4 carbon atoms, a halogen atom, a fluoroalkyl group having from 1 to 4 carbon atoms and at least one fluorine atom, a hydroxy group, an alkoxy group having from 1 to 4 carbon atoms, a fluoroalkoxy group having from 1 to 4 carbon atoms and at least one fluorine atom, an alkylthio group having from 1 to 4 carbon atoms, a fluoroalkylthio group having from 1 to 4 carbon atoms and at least one fluorine atom, an amino group, an alkanoyl group having from 1 to 5 carbon atoms, a fluoroalkanoyl group having from 2 to 5 carbon atoms and at least one fluorine atom, an alkoxycarbonyl group having from 2 to 5 carbon atoms, a carbamoyl group, a cyano group, a nitro group, an alkanesulfonyl group having from 1 to 4 carbon atoms, a fluoroalkanesulfonyl group having from 1 to 4 carbon atoms and at least one fluorine atom, or a sulfamoyl group;
- R₂: represents hydrogen or an alkanoyl group having from 2 to 6 carbon atoms, a substituted alkanoyl group which has from 2 to 6 carbon atoms and which is substituted by at least one substituent selected from the group consisting of substituents A', defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 7 carbon atoms, a substituted cycloalkylcarbonyl group which has from 4 to 7 carbon atoms and which is substituted by at least one substituent selected from the group consisting of substituents A', defined below or a substituted benzoyl group having at least one fluorine substituent;
- R₃: represents a hydrogen atom, a hydroxy group, an alkoxy group having from 1 to 4 carbon atoms, an alkoxymethoxy group in which the alkoxy part has from 1 to 4 carbon atoms, an alkanoyloxymethoxy group in which the alkanoyl part has from 1 to 5 carbon atoms, a benzyloxy group which is unsubstituted or is substituted by at least one substituent selected from the group consisting of substituents D', defined below, an alkanoyloxy group having from 1 to 18 carbon atoms, an alkenoyloxy group having 3 or 4 carbon atoms, a cycloalkylcarbonyloxy group having from 4 to 7 carbon atoms, a benzoyloxy group which is unsubstituted or is substituted by at least one substituent selected from the group consisting of substituents D', defined below, an alkoxycarbonyloxy group having from 2 to 5 carbon atoms, a benzyloxycarbonyloxy group which is unsubstituted or is substituted by at least one substituent selected from the group consisting of substituents D', defined below, a phthalidyloxy group, a (5-methyl-2-oxo-1,3-dioxolen-4-yl)methoxy group, a (5-phenyl-2-oxo-1,3-dioxolen-4-yl)methoxy group, a group of formula -NR^{a}R^{b}, wherein R^{a} and R^{b} are independently selected from the group consisting of hydrogen atoms, methyl groups and ethyl groups or R^{a} represents a hydrogen atom and R^{b} represents an alkanoyloxymethyl group in which the alkanoyl part has from 1 to 5 carbon atoms; a benzylamino group, an alkanoylamino group having from 1 to 18 carbon atoms, an alkenoylamino group having 3 or 4 carbon atoms, a cycloalkylcarbonylamino group having 6 or 7 carbon atoms, a benzoylamino group which is unsubstituted or is substituted by at least one substituent selected from the group consisting of substituents D', defined below, an alkoxycarbonylamino group having from 2 to 5 carbon atoms or a benzyloxycarbonylamino group which is unsubstituted or is substituted by at least one substituent selected from the group consisting of substituents D', defined below;
- said substituents A': are selected from the group consisting of fluorine atoms, chlorine atoms, hydroxy groups, methoxy groups, ethoxy groups and cyano groups; and
- said substituents D': are selected from the group consisting of fluorine atoms, chlorine atoms, methyl groups and methoxy groups
in formula III
the substituted position of the group of the formula 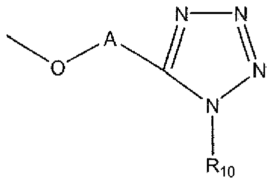 is 4-,7- or 8-position of the carbostyril skeleton
in formula IV
- R₁₃ and R₁₄: are each a benzylamino group of structural formulae II and
- R₁₁ and R₁₂: are selected from 2- methoxyethoxy, propoxy, 2-hydroxypropoxy, diethanolamino, solketalo, chloro, 2-hydroxyethoxy and 3-hydroxypropoxy
in formula V
- R₅' and R₆': are both hydrogen
for the use in the treatment and/or prevention of Laminitis in Ungulates.

Another embodiment of the invention is a compound or a tautomer thereof, or a pharmaceutically acceptable salt thereof, selected from the group comprising

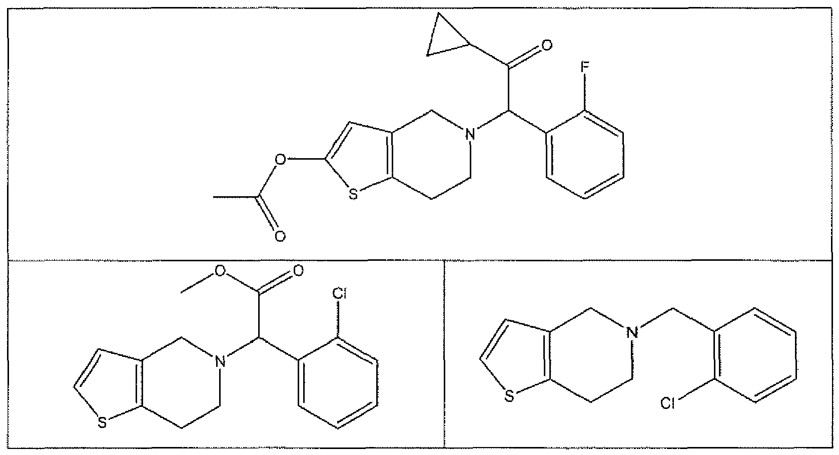

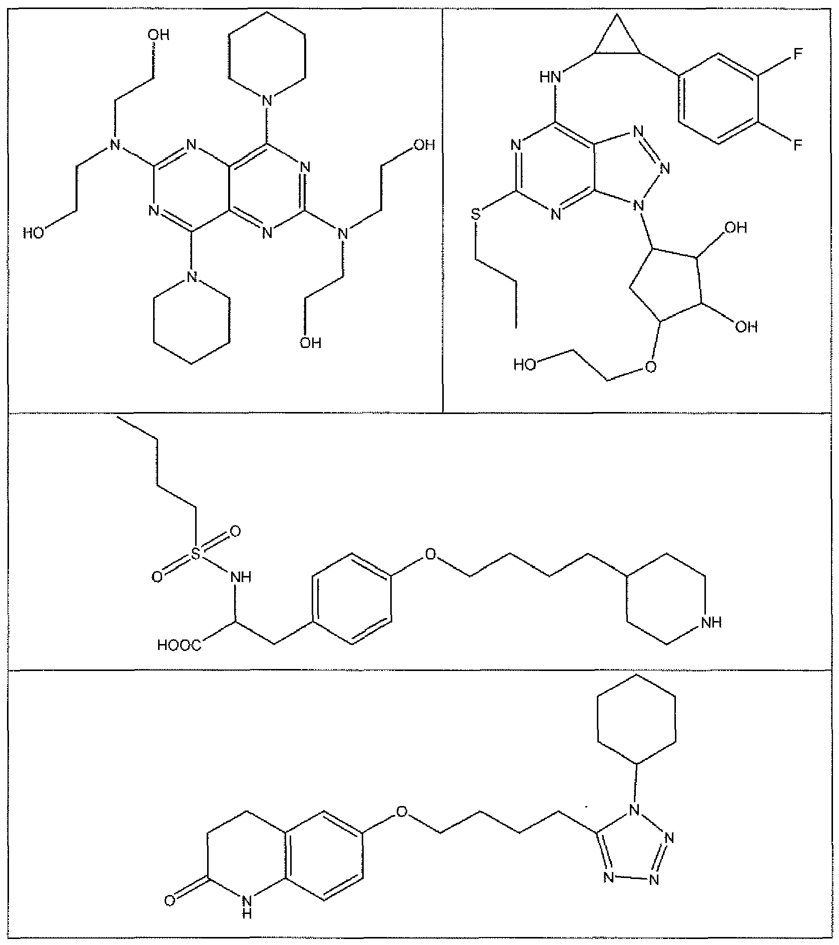

for the use in the treatment and/or prevention of Laminitis in Ungulates.

Another embodiment of the invention is a compound or a tautomer thereof, or a pharmaceutically acceptable salt with the following structure 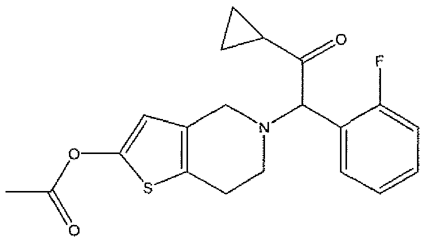 for the use in the treatment and/or prevention of Laminitis in Ungulates.

Another embodiment of the invention is a compound or a tautomer thereof, or a pharmaceutically acceptable salt with the following structure 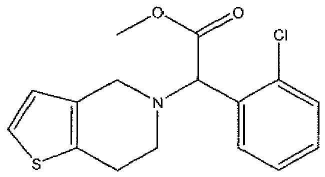 for the use in the treatment and/or prevention of Laminitis in Ungulates.

Another embodiment of the invention is a compound or a tautomer thereof, or a pharmaceutically acceptable salt with the following structure 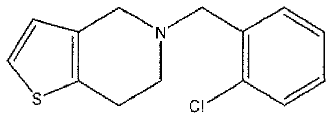 for the use in the treatment and/or prevention of Laminitis in Ungulates.

Another embodiment of the invention is a compound or a tautomer thereof, or a pharmaceutically acceptable salt with the following structure 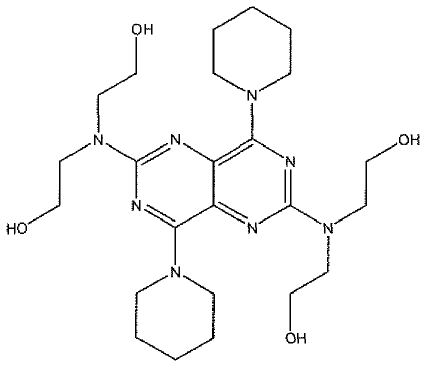 for the use in the treatment and/or prevention of Laminitis in Ungulates.

Another embodiment of the invention is a compound or a tautomer thereof, or a pharmaceutically acceptable salt with the following structure 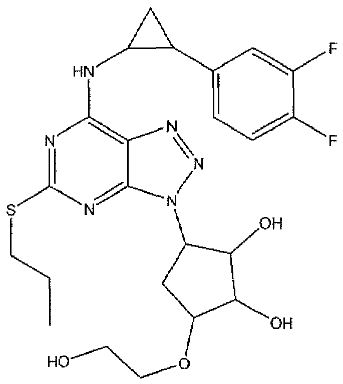 for the use in the treatment and/or prevention of Laminitis in Ungulates.

Another embodiment of the invention is a compound or a tautomer thereof, or a pharmaceutically acceptable salt with the following structure 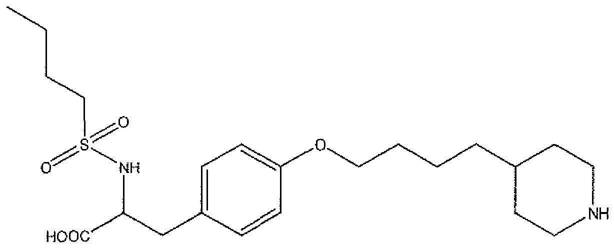 for the use in the treatment and/or prevention of Laminitis in Ungulates.

Another embodiment of the invention is a compound or a tautomer thereof, or a pharmaceutically acceptable salt with the following structure 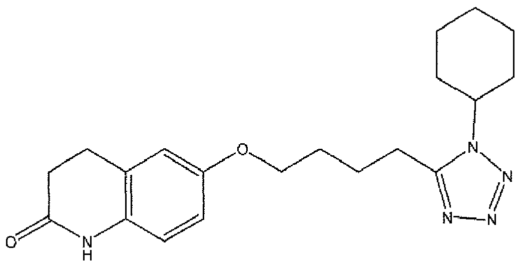 for the use in the treatment and/or prevention of Laminitis in Ungulates.

Another embodiment of the invention is a compound of formulae I - V or a tautomer thereof, or a pharmaceutically acceptable salt in combination with acetylsalicylic acid for the use in the treatment and/or prevention of Laminitis in Ungulates.

Another embodiment of the invention is a compound of formulae I - V or a tautomer thereof, or a pharmaceutically acceptable salt in the dosage of 5 - 100 mg in combination with 100 - 1000 mg acetylsalicylic acid per 60 kg given twice a day for the use in the treatment and/or prevention of Laminitis in Ungulates.

Another embodiment of the invention is a compound of formulae I - V or a tautomer thereof, or a pharmaceutically acceptable salt in the dosage of 5 - 100 mg in combination with 100 - 1000 mg acetylsalicylic acid per 60 kg given twice a day and an initial dosage of 10 - 200 mg in combination with 200 - 2000 mg acetylsalicylic acid per 60 kg for the use in the treatment and/or prevention of Laminitis in Ungulates.

Another embodiment of the invention is a compound or a tautomer thereof, or a pharmaceutically acceptable salt with the following structure 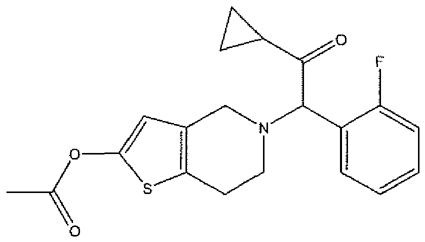 in the dosage of 10 mg in combination with 500 mg acetylsalicylic acid per 60 kg given twice a day for the use in the treatment and/or prevention of Laminitis in Ungulates.

Another embodiment of the invention is a compound or a tautomer thereof, or a pharmaceutically acceptable salt with the following structure 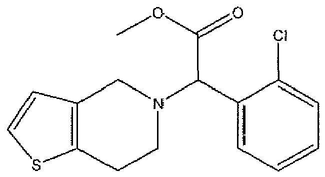 in the dosage of 75 mg in combination with 500 mg acetylsalicylic acid per 60 kg given twice a day for the use in the treatment and/or prevention of Laminitis in Ungulates.

Another embodiment of the invention is a compound of formulae I - V or a tautomer thereof, or a pharmaceutically acceptable salt for the use in the treatment and/or prevention of laminitis in even-toed and odd-toed Ungulates, preferred for equidae, bovinae, suidae, deer, ovis, capra, especially preferred for equidae.

Another embodiment of the invention is a platelet aggregation inhibitor for the use in the treatment and/or prevention of laminitis in even-toed and odd-toed Ungulates, preferred for equidae, bovinae, suidae, deer, ovis, capra, especially preferred for equidae.

Another embodiment of the invention is a compound of formulae I - V or a tautomer thereof, or a pharmaceutically acceptable salt for the use in the treatment and/or prevention of laminitis in even-toed and odd-toed Ungulates, preferred for bovinae, especially preferred for pregnant bovinae, especially for pregnant bovinae from 35 days before calving and to 70 days after calving.

Another embodiment of the invention is a platelet aggregation inhibitor for the use in the treatment and/or prevention of of laminitis in even-toed and odd-toed Ungulates, preferred for bovinae, especially preferred for pregnant bovinae, especially preferred for pregnant bovinae from 35 days before calving to 70 days after calving.

Another embodiment of the invention is animal feed comprising a platelet aggregation inhibitor.

Another embodiment of the invention is animal feed comprising a compound or a tautomer thereof, or a pharmaceutically acceptable salt thereof selected from the group comprising

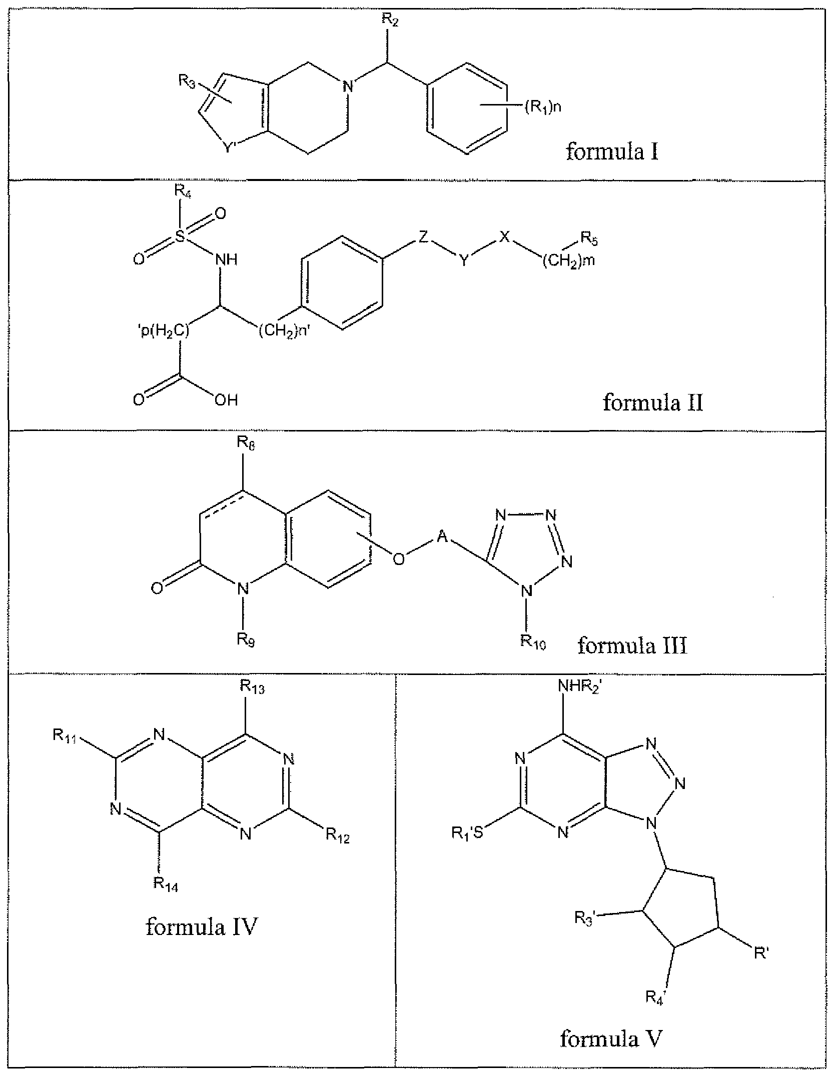

wherein
in formula I
- Y': is NH, O or S;
- R₁: represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl group;
- R₂: represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
- R₃: represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxolen-4yl)methoxy group; a (5-phenyl-2-oxo-1,3-dioxolen-4-yl)methoxy group; a group of formula -NR^{a} R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
- n: is an integer from 1 to 5, and, when n is an integer from 2 to 5, the groups represented by R₁ may be the same as or different from each other;
- said substituents A: are selected from the group consisting of halogen atoms, hydroxy groups, alkoxy groups having from 1 to 4 carbon atoms and cyano groups;
- said substituents B: are selected from the group consisting of alkyl groups having from 1 to 4 carbon atoms, halogen atoms and alkoxy groups having from 1 to 4 carbon atoms;
- said substituents C: are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom;
in formulae II
- R₄: is aryl, C₁₋₁₀ alkyl or C₄₋₁₀ aralkyl,
- Z: is a bond or O, -NHC(O)-, -C(O)NH-, C₁₋₅ straight or branched alkyl,
- m: is an integer from 0 to 6
- R₅: is
a) a six membered saturated heterocyclic ring containing one or two heteroatoms wherein said heteroatoms are N or O and wherein said heterocyclic ring is optionally substituted by C₁₋₃ alkyl,
   or
c) NR₆R₇, wherein R₆ and R₇ are independently hydrogen or C₁₋₁₀ alkyl
- X and Y: are independently C₁₋₁₀ alkyl or cycloalkyl, -NHC(O)- or -C(O)NH-
- n': is one or two
- p': is zero or one
in formula III
- R₈: is a hydrogen atom, a lower alkyl group or a group of the formula

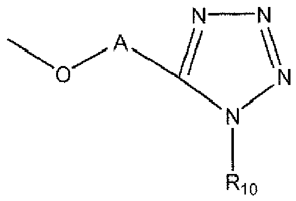

- R₉: is a hydrogen atom, a lower alkyl group, a lower alkenyl group, a lower alkanoyl group, a benzoyl group or a phenyl-C.sub.1-4 alkyl group;
- R₁₀: is a lower alkyl group, a C₃₋₈ cycloalkyl group, a C₃₋₈ cycloalkyl-C₁₋₄ alkyl group, a phenyl group or a phenyl-C₁₋₄ alkyl group;
- A: is a lower alkylene group,
the carbon-carbon bond between 3- and 4-positions in the carbostyril skeleton is either single or double bond; and the substituted position of the group represented by the formula, 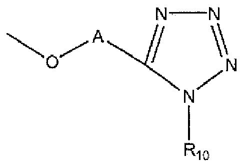 in the carbostyril skeleton is either 4-, 5-, 6-, 7- or 8-position provided that only one such group of the formula can be substituted in the whole carbostyril skeleton, thus when R⁸ in 4-position is 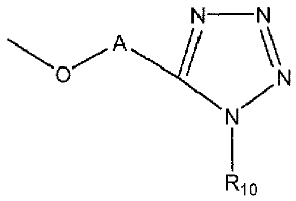 then 5-, 6-, 7- or 8-position will have no such substituted group; furthermore, the phenyl group in the above-mentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy. in formula IV
- R₁₁: is chloro and R₁₂ is diethanolamino, or
- R₁₁/R₁₂: are identical and are selected from allyl, halo, diethanolamino, solketalo and a group having the formula -O-R_{z} or -NHR_{z},
- R_{z}: is selected from alkyl, hydroxyalkyl, alkoxyalkyl, dialkoxyalkyl and 2-oxo-alkyl wherein the or each alkyl and/or alkoxy moiety has less than six carbon atoms, and
- R₁₃/R₁₄: are identical and are selected from piperidino, N-tetrahydroisoquinolyl, and a benzylamino group having the following structural formula: 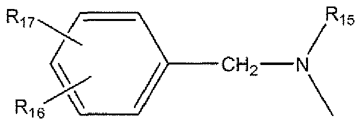 where
R₁₅ is H, or an optionally substituted alkyl or benzyl group, and
R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
- R₁': is a C₁₋₆ alkyl, C₂₋₆ alkenyl, C₂₋₆ alkenyl, C₃₋₈-cycloalkyl or a phenyl group, each group being optionally substituted by one or more substituents selected
- R₂': from halogen, OR₈', NR₉' R₁₀', SR₁₁' or C₁₋₆ alkyl (itself optionally substituted by one or more halogen atoms); is C₁₋₈ alkyl optionally substituted by one or more substituents selected from halogen, OR₈, NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂, C(O)R₈', OR₈', SR₁₁', NR₁₂'R₁₃', a fused 5- or 6- membered saturated ring containing one or two oxygen atoms, phenyl or C₁₋₆-alkyl the latter two groups being optionally substituted by OR₈', NR₉'R₁₀' or one or more halogen atoms;
- R₃'/R₄': one of them is hydroxy and the other is hydrogen, hydroxy or NR₉'R₁₀';
- R': is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅'R₆')ⱼR₁₄' where R₅' and R₆' are as defined above, j is 1 to 3 and R₁₄' is COOH, OR₁₅', NR₁₆'R₁₇' or CONR₁₆'R₁₇'; or R' is a C₁₋₄ alkyl or C₂₋₄ alkenyl group, each or which is substituted by one or more groups selected from =S, =O =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃' are independently hydrogen, C₁₋₄ alkyl or phenyl; R₂₀' is OR₂₄' or NR₂₅ 'R₂₆', where R₂₄' is hydrogen, C₁₋₄ alkyl or phenyl, and R₂₅' and R₂₆' are independently hydrogen, C₁₋₄ alkyl, aryl, C₁₋₆ acyl, arylsulphonyl or arylcarbonyl);
- R₈': is hydrogen, C₁₋₆ alkyl optionally substituted by halogen or R₈' is phenyl optionally substituted by one or more substituents selected from halogen, NO₂, C(O)R₆', OR₆', SR₉', NR₁₀'R₁₁';
- R₉'/R₁₀'^{/}R₁₁': are independently hydrogen or C₁₋₆ alkyl;
- R₁₂'^{/}R_{13'}: are independently hydrogen, C₁₋₆ alkyl, acyl, alkyl sulfonyl optionally substituted by halogen, or phenyl sulfonyl optionally substituted by C₁-C₄ alkyl; and
- R₁₅'/R₁₆'/R₁₇': are independently hydrogen C₁₋₆ alkyl.

Another embodiment of the invention is animal feed comprising a compound or a tautomer thereof, or a pharmaceutically acceptable salt thereof selected from the group comprising

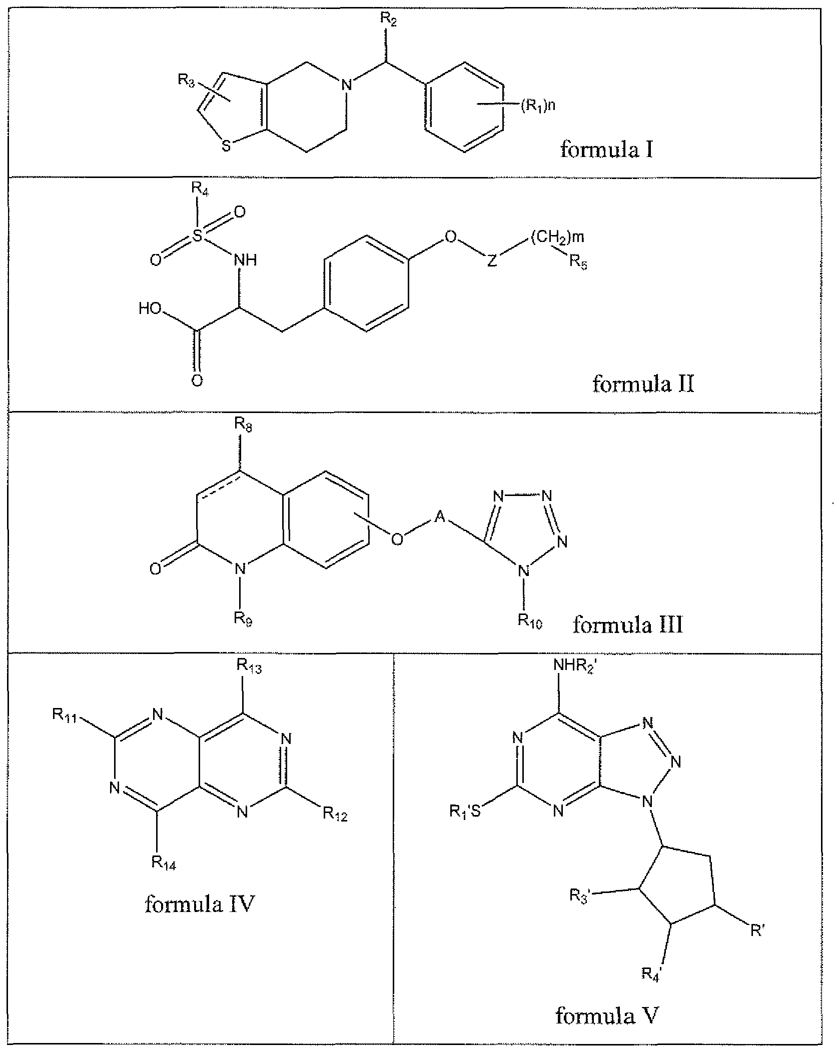

wherein
in formula I
- R₁: represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl group;
- R₂: represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
- R₃: represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxolen-4yl)methoxy group; a (5-phenyl-2-oxo-1,3-dioxolen-4-yl)methoxy group; a group of formula -NR^{a}R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
- n: is an integer from 1 to 5, and, when n is an integer from 2 to 5, the groups represented by R₁ may be the same as or different from each other;
- said substituents A: are selected from the group consisting of halogen atoms, hydroxy groups, alkoxy groups having from 1 to 4 carbon atoms and cyano groups;
- said substituents B: are selected from the group consisting of alkyl groups having from 1 to 4 carbon atoms, halogen atoms and alkoxy groups having from 1 to 4 carbon atoms;
- said substituents C: are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom;
in formula II
- R₄: is aryl, C₁₋₁₀ alkyl or C₄₋₁₀ aralkyl,
- Z: is a bond or O, -NHC(O)-, -C(O)NH-, C₁₋₅ straight or branched alkyl,
- m: is an integer from 2 to 6
- R₅: is
a) a six membered saturated heterocyclic ring containing one or two heteroatoms wherein said heteroatoms are N or O and wherein said heterocyclic ring is optionally substituted by C₁₋₃ alkyl,
   or
b) NR₆R₇, wherein R₆ and R₇ are independently hydrogen or C₁₋₁₀ alkyl
in formula III
- R₈: is a hydrogen atom, a lower alkyl group or a group of the formula

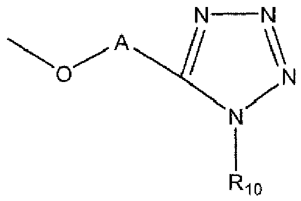

- R₉: is a hydrogen atom, a lower alkyl group, a lower alkenyl group, a lower alkanoyl group, a benzoyl group or a phenyl-C.sub.1-4 alkyl group;
- R₁₀: is a lower alkyl group, a C₃₋₈ cycloalkyl group, a C₃₋₈ cycloalkyl-C₁₋₄ alkyl group, a phenyl group or a phenyl-C₁₋₄ alkyl group;
- A: is a lower alkylene group,
the carbon-carbon bond between 3- and 4-positions in the carbostyril skeleton is either single or double bond; and the substituted position of the group represented by the formula, 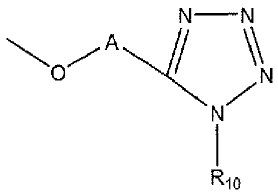 in the carbostyril skeleton is either 4-, 5-, 6-, 7- or 8-position provided that only one such group of the formula can be substituted in the whole carbostyril skeleton, thus when R⁸ in 4-position is 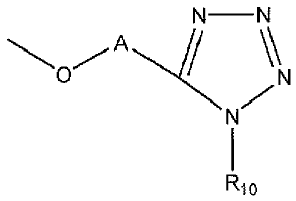 then 5-, 6-, 7- or 8-position will have no such substituted group; furthermore, the phenyl group in the above-mentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy. in formula IV
- R₁₁: is chloro and R₁₂ is diethanolamino, or
- R₁₁/R₁₂: are identical and are selected from allyl, halo, diethanolamino, solketalo and a group having the formula -O-R_{z} or -NHR_{Z},
- R_{Z}: is selected from alkyl, hydroxyalkyl, alkoxyalkyl, dialkoxyalkyl and 2-oxo-alkyl wherein the or each alkyl and/or alkoxy moiety has less than six carbon atoms, and
- R₁₃/R₁₄: are identical and are selected from piperidino, N-tetrahydroisoquinolyl, and a benzylamino group having the following structural formula: 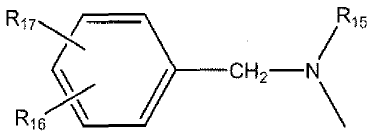 where
R₁₅ is H, or an optionally substituted alkyl or benzyl group, and
R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
- R₁': is a C₁₋₆ alkyl, C₂₋₆ alkenyl, C₂₋₆ alkenyl, C₃₋₈-cycloalkyl or a phenyl group, each group being optionally substituted by one or more substituents selected from halogen, OR₈', NR₉' R₁₀', SR₁₁' or C₁₋₆ alkyl (itself optionally substituted by one or more halogen atoms);
- R₂': is C₁₋₈ alkyl optionally substituted by one or more substituents selected from halogen, OR₈', NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂, C(O)R₈', OR₈', SR₁₁', NR₁₂'R₁₃', a fused 5- or 6- membered saturated ring containing one or two oxygen atoms, phenyl or C₁₋₆-alkyl the latter two groups being optionally substituted by OR₈', NR₉'R₁₀' or one or more halogen atoms;
- R₃'/R₄': one of them is hydroxy and the other is hydrogen, hydroxy or NR₉'R₁₀';
- R': is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅'R₆')ⱼR₁₄' where R₅' and R₆' are as defined above, j is 1 to 3 and R₁₄' is COOH, OR₁₅', NR₁₆'R₁₇' or CONR₁₆'R₁₇'; or R' is a C₁₋₄ alkyl or C₂₋₄ alkenyl group, each or which is substituted by one or more groups selected from =S, =O, =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃' are independently hydrogen, C₁₋₄ alkyl or phenyl; R₂₀' is OR₂₄' or NR₂₅'R₂₆', where R₂₄' is hydrogen, C₁₋₄ alkyl or phenyl, and R₂₅' and R₂₆' are independently hydrogen, C₁₋₄ alkyl, aryl, C₁₋₆ acyl, arylsulphonyl or arylcarbonyl);
- R₈': is hydrogen, C₁₋₆ alkyl optionally substituted by halogen or R₈' is phenyl optionally substituted by one or more substituents selected from halogen, NO₂, C(O)R₆', OR₆', SR₉', NR₁₀'R₁₁';
- R₉'/R₁₀^{'/}R₁₁': are independently hydrogen or C₁₋₆ alkyl;
- R₁₂'^{/}R_{13'}: are independently hydrogen, C₁₋₆ alkyl, acyl, alkyl sulfonyl optionally substituted by halogen, or phenyl sulfonyl optionally substituted by C₁₋C₄ alkyl; and
- R₁₅'/R₁₆'/R₁₇': are independently hydrogen C₁₋₆ alkyl.

Another embodiment of the invention is animal feed comprising a compound or a tautomer thereof, or a pharmaceutically acceptable salt thereof
wherein
in formula I
- R₁: represents a hydrogen atom, an alkyl group having from 1 to 4 carbon atoms, a halogen atom, a fluoroalkyl group having from 1 to 4 carbon atoms and at least one fluorine atom, a hydroxy group, an alkoxy group having from 1 to 4 carbon atoms, a fluoroalkoxy group having from 1 to 4 carbon atoms and at least one fluorine atom, an alkylthio group having from 1 to 4 carbon atoms, a fluoroalkylthio group having from 1 to 4 carbon atoms and at least one fluorine atom, an amino group, an alkanoyl group having from 1 to 5 carbon atoms, a fluoroalkanoyl group having from 2 to 5 carbon atoms and at least one fluorine atom, an alkoxycarbonyl group having from 2 to 5 carbon atoms, a carbamoyl group, a cyano group, a nitro group, an alkanesulfonyl group having from 1 to 4 carbon atoms, a fluoroalkanesulfonyl group having from 1 to 4 carbon atoms and at least one fluorine atom, or a sulfamoyl group
in formula II
- R₄: is aryl, C₁₋₁₀ alkyl or C₄₋₁₀ aralkyl;
- R₅: is
a) a six membered saturated heterocyclic ring containing one or two heteroatoms wherein said heteroatoms are N,
   or
b) NR₆R₇, wherein R₆ and R₇ are independently hydrogen or C₁₋₁₀ alkyl
- Z: is a bond or O, -NHC(O)-, or-C(O)NH-
- m: is an integer from 2 to 6
in formula III
the substituted position of the group of the formula 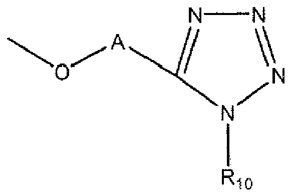 is 5 or 6-position of the carbostyril skeleton
in formula IV
all alkyl groups when present as such, or as a moiety m other groups such as alkoxy in R11-R17 and Rx, Ry and Rz, are lower alkyl groups containing 1-5 carbon atoms
in formula V
- R₁': is C₁₋₄ alkyl or phenyl substituted by trifluoromethyl.

Another embodiment of the invention is animal feed comprising a compound or a tautomer thereof, or a pharmaceutically acceptable salt thereof
wherein
in formula I
- R₂: represents hydrogen or an alkanoyl group having from 2 to 6 carbon atoms, a substituted alkanoyl group which has from 2 to 6 carbon atoms and which is substituted by at least one substituent selected from the group consisting of substituents A', defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group, a cycloalkylcarbonyl group having from 4 to 7 carbon atoms, a substituted cycloalkylcarbonyl group which has from 4 to 7 carbon atoms and which is substituted by at least one substituent selected from the group consisting of substituents A', defined below of a substituted benzoyl group having at least one fluorine substituent; and
- said substituents A': are selected from the group consisting of fluorine atoms, chlorine atoms, hydroxy groups, methoxy groups, ethoxy groups and cyano groups
in formula II
- R₄: is C₁₋₁₀ alkyl;
- R₅: is a six membered saturated heterocyclic ring containing one heteroatom wherein said heteroatom is N;
- Z: is a bond or O
- m: is an integer from 2 to 6
in formula III
- R₁₀: is a C₃₋₈ cycloalkyl group or a C₃₋₈ cycloalkyl-C₁₋₄ alkyl group
in formula IV
- R₁₃ and R₁₄: are piperidino, and
- R₁₁ and R₁₂: are each selected from allyl, methoxy, ethoxy, n-propoxy, iso-propoxy, iso-butoxy, 3-methylbutoxy, 2-oxo-n-propoxy, 2,2-diethoxy-n-propoxy, (2-methoxy-1-methyl) ethoxy, 2- methoxyethoxy, 2-hydroxypropoxy, 2-hydroxyethoxy and 3- hydroxypropoxy
in formula V
- R₂': is butyl or cyclopropyl optionally substituted by phenyl, the phenyl group itself being optionally substituted by one or more halogen, C₃₋₈ alkyl, phenoxy or phenyl groups.

Another embodiment of the invention is animal feed comprising a compound or a tautomer thereof, or a pharmaceutically acceptable salt thereof
wherein
in formula I
- R₃: represents a hydrogen atom, a hydroxy group, an alkoxy group having from 1 to 4 carbon atoms, an alkoxymethoxy group in which the alkoxy part has from 1 to 4 carbon atoms, an alkanoyloxymethoxy group in which the alkanoyl part has from 1 to 5 carbon atoms, a benzyloxy group which is unsubstituted or is substituted by at least one substituent selected from the group consisting of substituents D', defined below, an alkanoyloxy group having from 1 to 18 carbon atoms, an alkenoyloxy group having 3 or 4 carbon atoms, a cycloalkylcarbonyloxy group having from 4 to 7 carbon atoms, a benzoyloxy group which is unsubstituted or is substituted by at least one substituent selected from the group consisting of substituents D', defined below, an alkoxycarbonyloxy group having from 2 to 5 carbon atoms, a benzyloxy. carbonyloxy group which is unsubstituted or is substituted by at least one substituent selected from the group consisting of substituents D', defined below, a phthalidyloxy group, a (5-methyl-2-oxo-1,3-dioxolen-4-yl)methoxy group, a (5-phenyl-2-oxo-1,3-dioxolen-4-yl)methoxy group, a group of formula -NR^{a}R^{b}, wherein R^{a} and R^{b} are independently selected from the group consisting of hydrogen atoms, methyl and ethyl groups or R^{a} represents a hydrogen atom and R^{b} represents an alkanoyloxymethyl group in which the alkanoyl part has from 1 to 5 carbon atoms; a benzylamino group, an alkanoylamino group having from 1 to 18 carbon atoms, an alkenoylamino group having 3 or 4 carbon atoms, a cycloalkylcarbonylamino group having 6 or 7 carbon atoms, a benzoylamino group which is unsubstituted or is substituted by at least one substituent selected from the group consisting of substituents D', defined below, an alkoxycarbonylamino group having from 2 to 5 carbon atoms or a benzyloxycarbonylamino group which is unsubstituted or is substituted by at least one substituent selected from the group consisting of substituents D', defined below;

- said substituents D': are selected from the group consisting of fluorine atoms, chlorine atoms, methyl groups and methoxy groups
in formula II
- R₅: is a six membered saturated heterocyclic ring containing one heteroatom wherein said heteroatom is N;
- R₄: is -(CH₂)(CH₂)(CH₂)(CH₃);
- Z: is a bond or O
- m: is 4
in formula III
- R¹⁰: is a phenyl-C₁₋₄ alkyl group which may have substituted group(s) selected from the group consisting of lower alkoxy, lower alkyl, halogen, di-lower alkylamino, nitro, and lower alkenedioxy, a phenyl group which may have substituted group(s) selected from the group consisting of lower alkoxy, lower alkyl, halogen, di-lower alkylamino, nitro, and lower alkenedioxy or a lower alkyl group
in formula IV
- R₁₃ and R₁₄: are N-tetrahydroisoquinolyl and
- R₁₁ and R₁₂: are each selected from diethanolamino and chloro in formula V
- R₃' and R₄': are both hydroxy.

Another embodiment of the invention is animal feed comprising a compound or a tautomer thereof, or a pharmaceutically acceptable salt thereof
wherein
in formula I
- R₁: represents a hydrogen atom, an alkyl group having from I to 4 carbon atoms, a halogen atom, a fluoroalkyl group having from 1 to 4 carbon atoms and at least one fluorine atom, a hydroxy group, an alkoxy group having from 1 to 4 carbon atoms, a fluoroalkoxy group having from 1 to 4 carbon atoms and at least one fluorine atom, an alkylthio group having from 1 to 4 carbon atoms, a fluoroalkylthio group having from 1 to 4 carbon atoms and at least one fluorine atom, an amino group, an alkanoyl group having from 1 to 5 carbon atoms, a fluoroalkanoyl group having from 2 to 5 carbon atoms and at least one fluorine atom, an alkoxycarbonyl group having from 2 to 5 carbon atoms, a carbamoyl group, a cyano group, a nitro group, an alkanesulfonyl group having from 1 to 4 carbon atoms, a fluoroalkanesulfonyl group having from 1 to 4 carbon atoms and at least one fluorine atom, or a sulfamoyl group;
- R₂: represents hydrogen or an alkanoyl group having from 2 to 6 carbon atoms, a substituted alkanoyl group which has from 2 to 6 carbon atoms and which is substituted by at least one substituent selected from the group consisting of substituents A', defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 7 carbon atoms, a substituted cycloalkylcarbonyl group which has from 4 to 7 carbon atoms and which is substituted by at least one substituent selected from the group consisting of substituents A', defined below or a substituted benzoyl group having at least one fluorine substituent;
- R₃: represents a hydrogen atom, a hydroxy group, an alkoxy group having from 1 to 4 carbon atoms, an alkoxymethoxy group in which the alkoxy part has from 1 to 4 carbon atoms, an alkanoyloxymethoxy group in which the alkanoyl part has from 1 to 5 carbon atoms, a benzyloxy group which is unsubstituted or is substituted by at least one substituent selected from the group consisting of substituents D', defined below, an alkanoyloxy group having from 1 to 18 carbon atoms, an alkenoyloxy group having 3 or 4 carbon atoms, a cycloalkylcarbonyloxy group having from 4 to 7 carbon atoms, a benzoyloxy group which is unsubstituted or is substituted by at least one substituent selected from the group consisting of substituents D', defined below, an alkoxycarbonyloxy group having from 2 to 5 carbon atoms, a benzyloxycarbonyloxy group which is unsubstituted or is substituted by at least one substituent selected from the group consisting of substituents D', defined below, a phthalidyloxy group, a (5-methyl-2-oxo-1,3-dioxolen-4-yl)methoxy group, a (5-phenyl-2-oxo-1,3-dioxolen-4-yl)methoxy group, a group of formula -NR^{a}R^{b}, wherein R^{a} and R^{b} are independently selected from the group consisting of hydrogen atoms, methyl groups and ethyl groups or R^{a} represents a hydrogen atom and R^{b} represents an alkanoyloxymethyl group in which the alkanoyl part has from 1 to 5 carbon atoms; a benzylamino group, an alkanoylamino group having from 1 to 18 carbon atoms, an alkenoylamino group having 3 or 4 carbon atoms, a cycloalkylcarbonylamino group having 6 or 7 carbon atoms, a benzoylamino group which is unsubstituted or is substituted by at least one substituent selected from the group consisting of substituents D', defined below, an alkoxycarbonylamino group having from 2 to 5 carbon atoms or a benzyloxycarbonylamino group which is unsubstituted or is substituted by at least one substituent selected from the group consisting of substituents D', defined below;
- said substituents A': are selected from the group consisting of fluorine atoms, chlorine atoms, hydroxy groups, methoxy groups, ethoxy groups and cyano groups; and
- said substituents D': are selected from the group consisting of fluorine atoms, chlorine atoms, methyl groups and methoxy groups
in formula III
the substituted position of the group of the formula 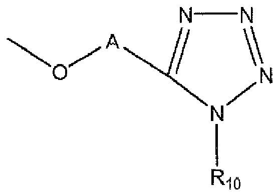 is 4-,7- or 8-position of the carbostyril skeleton
in formula IV
- R₁₃ and R₁₄: are each a benzylamino group of structural formulae II and
- R₁₁ and R₁₂: are selected from 2- methoxyethoxy, propoxy, 2-hydroxypropoxy, diethanolamino, solketalo, chloro, 2-hydroxyethoxy and 3-hydroxypropoxy
in formula V
- R₅' and R₆': are both hydrogen.

Another embodiment of the invention is animal feed comprising a compound or a tautomer thereof, or a pharmaceutically acceptable salt thereof, selected from the group comprising

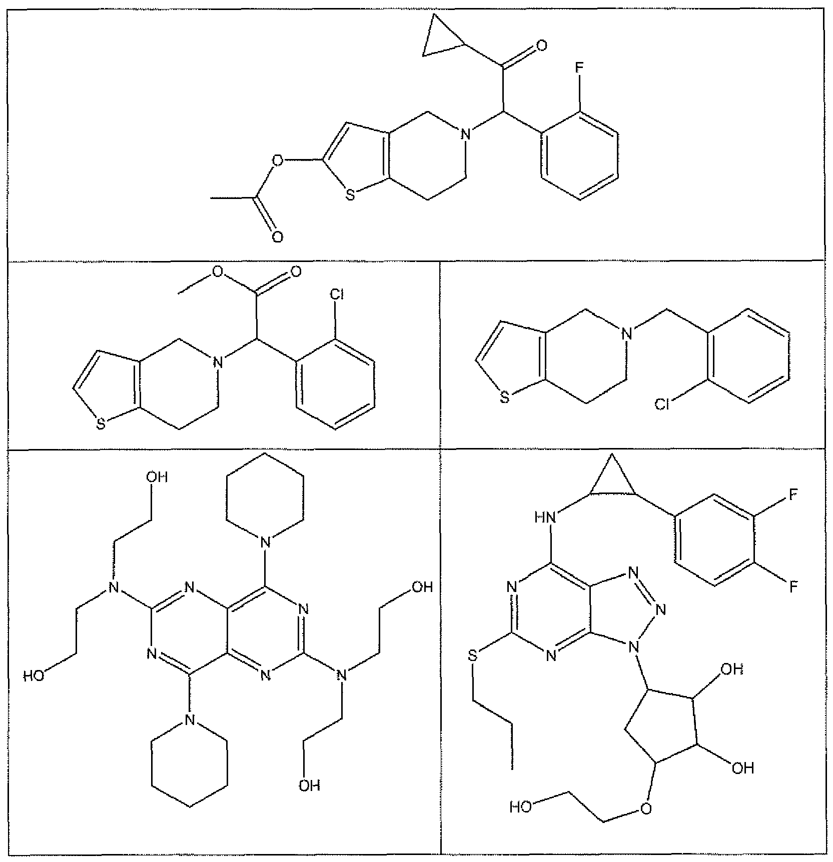

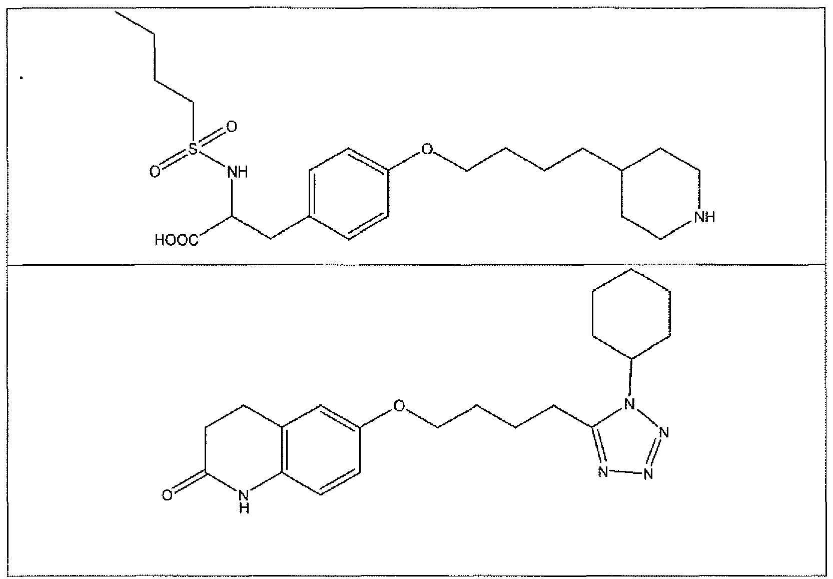

Another embodiment of the invention is animal feed comprising a compound or a tautomer thereof, or a pharmaceutically acceptable salt thereof with the following structure

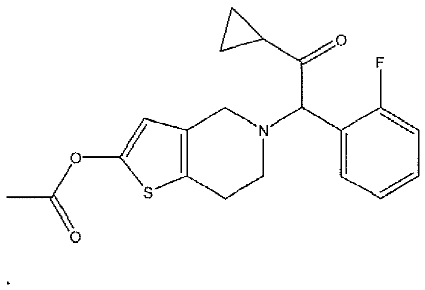

Another embodiment of the invention is animal feed comprising a compound or a tautomer thereof, or a pharmaceutically acceptable salt thereof with the following structure

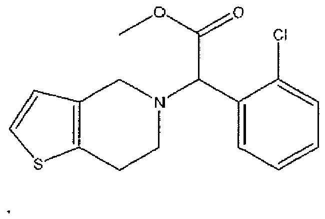

Another embodiment of the invention is animal feed comprising a compound or a tautomer thereof, or a pharmaceutically acceptable salt thereof with the following structure

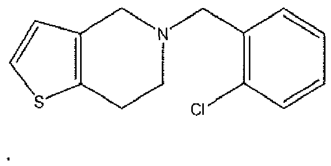

Another embodiment of the invention is animal feed comprising a compound or a tautomer thereof, or a pharmaceutically acceptable salt thereof with the following structure

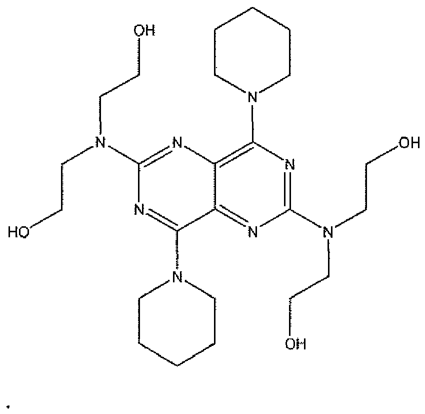

Another embodiment of the invention is animal feed comprising a compound or a tautomer thereof, or a pharmaceutically acceptable salt thereof with the following structure

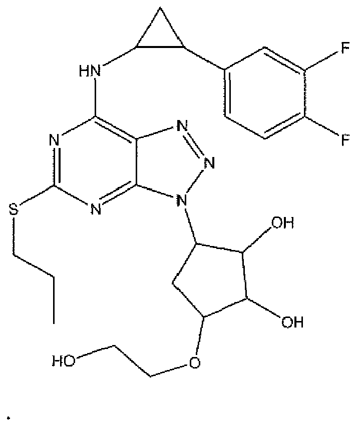

Another embodiment of the invention is animal feed comprising a compound or a tautomer thereof, or a pharmaceutically acceptable salt thereof with the following structure

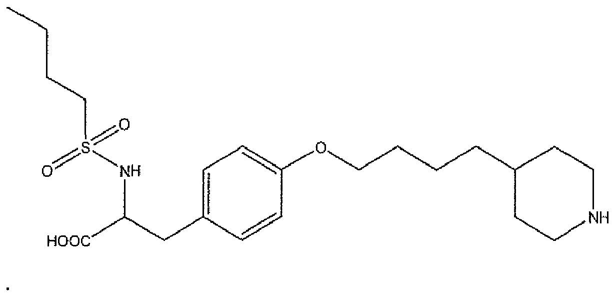

Another embodiment of the invention is animal feed comprising a compound or a tautomer thereof, or a pharmaceutically acceptable salt thereof with the following structure 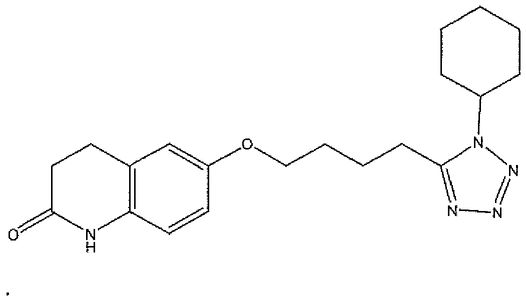 Another embodiment of the invention is animal feed comprising a compound or a tautomer thereof, or a pharmaceutically acceptable salt thereof in combination with acetylsalicylic acid. Another embodiment of the invention is animal feed comprising a compound or a tautomer thereof, or a pharmaceutically acceptable salt thereof in the dosage of 5 - 100 mg in combination with 100 -1000 mg acetylsalicylic acid per 60 kg given twice a day.

Another embodiment of the invention is animal feed comprising a compound or a tautomer thereof, or a pharmaceutically acceptable salt thereof in the dosage of 5 - 100 mg in combination with 100 - 1000 mg acetylsalicylic acid per 60 kg given twice a day and an initial dosage of 10 - 200 mg in combination with 200 - 2000 mg acetylsalicylic acid per 60 kg.

Another embodiment of the invention is animal feed comprising a compound or a tautomer thereof, or a pharmaceutically acceptable salt thereof with the following structure 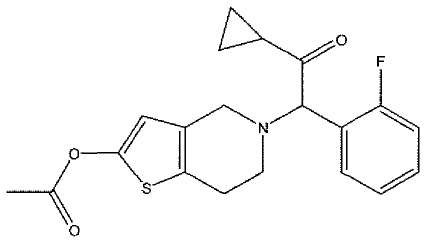 in the dosage of 10 mg in combination with 500 mg acetylsalicylic acid per 60 kg given twice a day.

Another embodiment of the invention is animal feed comprising a compound or a tautomer thereof, or a pharmaceutically acceptable salt thereof with the following structure 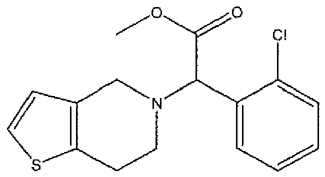 in the dosage of 75 mg in combination with 500 mg acetylsalicylic acid per 60 kg given twice a day.

Another embodiment of the invention is animal feed comprising a compound or a tautomer thereof, or a pharmaceutically acceptable salt thereof for the use in the treatment and/or prevention of even-toed and odd-toed Ungulates, preferred for equidae, bovinae, suidae, deer, ovis, capra, especially preferred for equidae.

Another embodiment of the invention is animal feed comprising a compound or a tautomer thereof, or a pharmaceutically acceptable salt thereof for the use in the treatment and/or prevention of even-toed and odd-toed Ungulates, preferred for bovinae, especially preferred for pregnant bovinae, especially preferred for pregnant bovinae from 35 days before calving to 70 days after calving.

### Concerning formula I:

In the compounds of the invention according to formulae I, where R₁ represents an alkyl group, this may be a straight or branched chain alkyl group having from 1 to 4 carbon atoms, and examples include the methyl, ethyl, propyl, isopropyl, butyl, isobutyl, sec-butyl and t-butyl groups. Of these, we prefer those alkyl groups having from 1 to 3 carbon atoms, more preferably the methyl and ethyl groups;

In the compounds of the invention according to formula I, where R₁ represents a halogen atom, this may be, for example, a fluorine, chlorine, iodine or bromine atom, and is preferably a fluorine or chlorine atom.

In the compounds of the invention according to formula I, where R₁ represents a haloalkyl group, the alkyl part may be any one of the alkyl groups exemplified above and may be substituted by one or more halogen (for example fluorine, chlorine, bromine or iodine) atoms. There is, in principle, no restriction on the number of halogen substituents on the alkyl group, this being limited only by the number of substitutable atoms. In general, however, from 1 to 5 halogen substituents are preferred, from 1 to 3 substituents being more preferred. Specific examples of such groups include the fluoromethyl, difluoromethyl, trifluoromethyl, chloromethyl, dichloromethyl, trichloromethyl, 2-fluoroethyl, 2-chloroethyl, 2-bromoethyl, 2-iodoethyl, 2,2,2-trichloroethyl, 2,2,2-trifluoroethyl, 2-fluoropropyl, 3-fluoropropyl, 3-chloropropyl, 2-fluorobutyl, 3-fluorobutyl, 4-chlorobutyl and 4-fluorobutyl groups. The fluorine-substituted and chlorine-substituted groups are preferred, the fluorine-substituted groups being more preferred. The fluoromethyl, difluoromethyl and trifluoromethyl groups are most preferred, especially the trifluoromethyl group.

In the compounds of the invention according to formula I, where R₁ represents an alkoxy group, this may be a straight or branched chain alkoxy group having from 1 to 4 carbon atoms, and examples include the methoxy, ethoxy, propoxy, isopropoxy, butoxy, isobutoxy, sec-butoxy and t-butoxy groups. Of these, we prefer those alkoxy groups having from 1 to 3 carbon atoms, more preferably the methoxy and ethoxy groups.

In the compounds of the invention according to formula I, where R₁ represents a haloalkoxy group, the alkoxy part may be any one of the alkoxy groups exemplified above and may be substituted by one or more halogen (for example fluorine, chlorine, bromine or iodine) atoms. There is, in principle, no restriction on the number of halogen substituents on the alkoxy group, this being limited only by the number of substitutable atoms. In general, however, from 1 to 5 halogen substituents are preferred, from 1 to 3 substituents being more preferred. Specific examples of such groups include the fluoromethoxy, difluoromethoxy, trifluoromethoxy, 2-fluoroethoxy, 2-chloroethoxy, 2-bromoethoxy, 2-iodoethoxy, 2,2,2-trichloroethoxy, 2,2,2-trifluoroethoxy, 2-fluoropropoxy, 3-fluoropropoxy, 3-chloropropoxy, 2-fluorobutoxy, 3-fluorobutoxy, 4-chlorobutoxy and 4-fluorobutoxy groups. The fluoroalkoxy groups are preferred. The fluoromethoxy, difluoromethoxy and trifluoromethoxy groups are most preferred, especially the trifluoromethoxy group.

In the compounds of the invention according to formula I, where R₁ represents an alkylthio group, this may be a straight or branched chain alkylthio group having from 1 to 4 carbon atoms, and examples include the methylthio, ethylthio, propylthio, isopropylthio, butylthio, isobutylthio, sec-butylthio and t-butylthio groups. Of these, we prefer those alkylthio groups having from 1 to 3 carbon atoms, more preferably the methylthio and ethylthio groups.

In the compounds of the invention according to formula I, where R₁ represents a haloalkylthio group, the alkylthio part may be any one of the alkylthio groups exemplified above and may be substituted by one or more halogen (for example fluorine, chlorine, bromine or iodine) atoms. There is, in principle, no restriction on the number of halogen substituents on the alkylthio group, this being limited only by the number of substitutable atoms. In general, however, from 1 to 5 halogen substituents are preferred, from 1 to 3 substituents being more preferred. Specific examples of such groups include the fluoromethylthio, difluoromethio, trifluoromethylthio, 2-fluoroethylthio, 2-chloroethylthio, 2-bromoethylthio, 2-iodoethylthio, 2,2,2-trichloroethylthio, 2,2,2-trifluoroethylthio, 2-fluoropropylthio, 3-fluoropropylthio, 3-chloropropylthio, 2-fluorobutylthio, 3-fluorobutylthio, 4-chlorobutylthio and 4-fluorobutylthio groups. The fluorine substituted groups are preferred. The fluoromethylthio, difluoromethylthio and trifluoromethylthio groups are most preferred, especially the trifluoromethylthio group.

In the compounds of the invention according to formula I, where R₁ represents an alkanoyl group, this has from 1 to 5 carbon atoms and may be a straight or branched chain group. Examples include the formyl, acetyl, propionyl, butyryl, isobutyryl, valeryl, isovaleryl and pivaloyl groups, of which the formyl and acetyl groups are preferred.

In the compounds of the invention according to formula I, where R₁ represents a haloalkanoyl group, this has from 2 to 5 carbon atoms and may be a straight or branched chain group. Examples include the fluoroacetyl, difluoroacetyl, trifluoroacetyl, chloroacetyl, trichloroacetyl, bromoacetyl, iodoacetyl, 3-fluoropropionyl, 4-fluorobutyryl and 5-fluorovaleryl groups. Of these, the fluorine substituted alkanoyl groups are preferred, the fluoroacetyl, difluoroacetyl and trifluoroacetyl groups being more preferred and the trifluoroacetyl group being most preferred.

In the compounds of the invention according to formula I, where R₁ represents an alkoxycarbonyl group, this may be a straight or branched chain alkoxycarbonyl group having from 2 to 5 carbon atoms, that is the alkoxy part has from 1 to 4 carbon atoms, and examples include the methoxycarbonyl, ethoxycarbonyl, propoxycarbonyl, isopropoxycarbonyl, butoxycarbonyl, isobutoxycarbonyl, sec-butoxycarbonyl and t-butoxycarbonyl groups. Of these, we prefer those alkoxycarbonyl groups having from 1 to 3 carbon atoms, more preferably the methoxycarbonyl and ethoxycarbonyl groups.

In the compounds of the invention according to formula I, where R₁ represents an alkanesulfonyl group, this may be a straight or branched chain alkanesulfonyl group having from 1 to 4 carbon atoms, and examples include the methanesulfonyl, ethanesulfonyl, propanesulfonyl, isopropanesulfonyl, butanesulfonyl, isobutanesulfonyl, sec butanesulfonyl and t-butanesulfonyl groups. Of these, we prefer those alkanesulfonyl groups having from 1 to 3 carbon atoms, more preferably the methanesulfonyl and ethanesulfonyl groups.

In the compounds of the invention according to formula I, where R₁ represents a haloalkanesulfonyl group, the alkanesulfonyl part may be any one of the alkanesulfonyl groups exemplified above and may be substituted by one or more halogen (for example fluorine, chlorine, bromine or iodine) atoms. There is, in principle, no restriction on the number of halogen substituents on the alkanesulfonyl group, this being limited only by the number of substitutable atoms. In general, however, from 1 to 5 halogen substituents are preferred, from 1 to 3 substituents being more preferred. Specific examples of such groups include the fluoromethanesulfonyl, difluoromethanesulfbnyl, trifluoromethanesulfonyl, dichloromethanesulfonyl, trichloromethanesulfonyl, 2-fluoroethanesulfonyl, 2-chloroethanesulfonyl 2-bromoethanesulfonyl, 2-iodoethanesulfonyl, 2,2,2-trichloroethanesulfonyl, 2,2,2-trifluoroethanesulfonyl, 2-fluoropropanesulfonyl, 3-fluoropropanesulfonyl, 3-chloropropanesulfonyl, 2-fluorobutanesulfonyl, 3-fluorobutanesulfonyl, 4-chlorobutanesulfonyl and 4-fluorobutanesulfonyl groups. The fluorine-substituted alkanesulfonyl and chlorine substituted alkanesulfonyl groups are preferred, the fluorine-substituted alkanesulfonyl groups being more preferred. The fluoromethanesulfonyl, difluoromethanesulfonyl and trifluoromethanesulfonyl groups are most preferred, especially the trifluoromethanesulfonyl group.

Of the above groups and atoms, we especially prefer that R₁ should represent: a hydrogen atom; an alkyl group having from 1 to 4 carbon atoms; a halogen atom; a fluorine substituted alkyl group having from 1 to 4 carbon atoms; a hydroxy group; an alkoxy group having from 1 to 4 carbon atoms; a fluorine-substituted alkoxy group having from 1 to 4 carbon atoms; an alkylthio group having from 1 to 4 carbon atoms; a fluorine-substituted alkylthio group having from 1 to 4 carbon atoms; an amino group; an alkanoyl group having from 1 to 5 carbon atoms; a fluorine-substituted alkanoyl group having from 2 to 5 carbon atoms; an alkoxycarbonyl group having from 2 to 5 carbon atoms; a carbamoyl group; a cyano group; a nitro group; an alkanesulfonyl group having from 1 to 4 carbon atoms; a fluorine-substituted alkanesulfonyl group having from 1 to 4 carbon atoms; or a sulfamoyl group.

More preferably R₁ represents: a hydrogen atom; a methyl group; an ethyl group; a halogen atom; a fluorine-substituted methyl group; a hydroxy group; a methoxy group; an ethoxy group; a fluorine-substituted methoxy group; a methylthio group; a fluorine-substituted methylthio group; a formyl group; an acetyl group; a fluorine-substituted acetyl group; a methoxycarbonyl group; an ethoxycarbonyl group; a propoxycarbonyl group; a carbamoyl group; a cyano group; a nitro group; a methanesulfonyl group; an ethanesulfonyl group; a fluorine-substituted methanesulfonyl group; or a sulfamoyl group.

Still more preferably R₁ represents: a halogen atom; a trifluoromethyl group; a hydroxy group; a difluoromethoxy group; a trifluoromethoxy group; a difluoromethylthio group; a trifluoromethylthio group; a formyl group; an acetyl group; a trifluoroacetyl group; a cyano group or a nitro group.

Most preferably R₁ represents: a fluorine atom, a chlorine atom or a trifluoromethyl group; especially a fluorine atom or a chlorine atom.

The number of the substituents, n, represented by R₁ is from 1 to 5, although the maximum may be lower than 5 in some cases if there is a problem of steric hindrance. Preferably n is from 1 to 3, and more preferably 1 or 2. The position of substitution by R₁ on the phenyl group is preferably para or ortho, more preferably ortho.

In the compounds of the invention according to formula I, where R₂ represents an alkanoyl group having from 1 to 10 carbon atoms, this may be a straight or branched chain group, and examples include the formyl, acetyl, propionyl, butyryl, isobutyryl, valeryl, isovaleryl, pivaloyl, hexanoyl, heptanoyl, octanoyl, nonanoyl and decanoyl groups, of which those groups having from 2 to 6 carbon atoms are preferred, especially the acetyl, propionyl and isobutyryl groups, of which the acetyl and propionyl groups are most preferred.

Those alkanoyl groups represented by R₂ and having from 2 to 10 carbon atoms may be substituted by one or more of substituents A, defined above.

In the compounds of the invention according to formula I, where R₂ represents an alkanoylcarbonyl group having from 2 to 10 carbon atoms, this may be of the form of -C(O)-O-C_{w} and w being an alkyl group of 1-9 carbon atoms and may be a straight or branched chain group.

Examples of such substituents A include: halogen atoms, such as the fluorine, chlorine, bromine and iodine atoms, hydroxyl groups; alkoxy groups having from 1 to 4 carbon atoms, such as those exemplified above in relation to R_{f}; and cyano groups.

In the case of these substituted groups, and all substituted groups referred to herein, there is no specific limitation on the number of the substituents, except such as may be imposed by the number of substitutable positions and possibly also by steric constraints. However, in general, from 1 to 3 such substituents are preferred.

Specific examples of such substituted alkanoyl groups include the fluoroacetyl, difluoroacetyl, trifluoroacetyl, chloroacetyl, trichloroacetyl, bromoacetyl, iodoacetyl, 3-fluoropropionyl, 3-cloropropionyl, 3-bromopropionyl, 3-iodopropionyl, 4-fluorobutyryl, 4-chlorobutyryl, 5-fluorovaleryl, hydroxyacetyl, 3-hydroxypropionyl, 4-hydroxybutyryl, 5-hydroxyvaleryl, methoxyacetyl, 3-methoxypropionyl, 4-methoxybutyryl, 5-methoxyvaleryl, ethoxyacetyl, 3-ethoxypropionyl, 4-ethoxybutyryl, 5-ethoxyvaleryl, cyanoacetyl, 3-cyanopropionyl, 4-cyanobutyryl and 5-cyanovaleryl groups, of which the fluoroacetyl, difluoroacetyl, trifluoroacetyl, chloroacetyl, 3-fluoropropionyl, 3-chloropropionyl, hydroxyacetyl, 3-hydroxypropionyl, methoxyacetyl, 3-methoxypropionyl, ethoxyacetyl, cyanoacetyl and 3-cyanopropionyl groups are more preferred. Still more preferred are the fluoroacetyl, difluoroacetyl, trifluoroacetyl, chloroacetyl, 3-fluoropropionyl, hydroxyacetyl, methoxyacetyl, ethoxyacetyl and cyanoacetyl groups. The most preferred groups are the fluoroacetyl, difluoroacetyl, trifluoroacetyl, chloroacetyl, 3-fluoropropionyl, hydroxyacetyl, methoxyacetyl and cyanoacetyl groups, especially the fluoroacetyl, difluoroacetyl and trifluoroacetyl groups.

In the compounds of the invention according to formula I, where R₂ represents an alkenoyl group having from 3 to 6 carbon atoms, this may be a straight or branched chain group, and examples include the acryloyl, methacryloyl, 2-butenoyl, 2-pentenoyl and 2-hexenoyl groups, of which the acryloyl and methacryloyl groups are preferred.

These alkenoyl groups may also be substituted by one or more of substituents A, defined and exemplified above. Specific examples of such substituted groups include the 3-fluoroacryloyl, 3-chloroacryloyl and 3-cyanoacryloyl groups, of which the 3-fluoroacryloyl group is particularly preferred.

In the compounds of the invention according to formula I, where R₂ represents a cycloalkylcarbonyl group, this has from 4 to 8 carbon atoms, that is the cycloalkyl group itself has from 3 to 7 ring carbon atoms. Examples of such groups include the cyclopropylcarbonyl, cyclobutylcarbonyl, cyclopentylcarbonyl, cyclohexyl carbonyl and cycloheptylcarbonyl groups, of which the cyclopropylcarbonyl and cyclobutylcarbonyl groups are particularly preferred.

These cycloalkylcarbonyl groups may also be substituted by one or more of substituents A, defined and exemplified above. Specific examples of such substituted groups include the 2-fluorocyclopropylcarbonyl, 2,2-difluoro cyclopropylcarbonyl, 2-chlorocyclopropylcarbonyl, 2-bromocyclopropylcarbonyl, 2-fluorocyclobutylcarbonyl, 2-chlorocyclobutylcarbonyl, 2-fluorocyclopentylcarbonyl, 2-chlorocyclopentyl carbonyl, 2-fluorocyclohexylcarbonyl, 2-chlorocyclohexylcarbonyl, 2-hydroxycyclopropylcarbonyl, 2-hydroxycyclobutylcarbonyl, 2-hydroxycyclopentylcarbonyl, 2-hydroxycyclohexylcarbonyl, 2-methoxycyclopropylcarbonyl, 2-methoxycyclobutylcarbonyl, 2-methoxycyclopentylcarbonyl, 2-methoxycyclohexylcarbonyl, 2-ethoxycyclopropylcarbonyl, 2-ethoxycyclobutylcarbonyl, 2-ethoxycyclopentylcarbonyl, 2-ethoxycyclohexylcarbonyl, 2-cyanocyclopropylcarbonyl, 2-cyanocyclobutylcarbonyl, 2-cyanocyclopentylcarbonyl and 2-cyanocyclohexylcarbonyl groups, of which the 2-fluorocyclopropylcarbonyl, 2,2-difluorocyclopropylcarbonyl, 2-chlorocyclopropylcarbonyl, 2-fluorocyclobutylcarbonyl, 2-chlorocyclobutylcarbonyl, 2 fluorocyclopentylcarbonyl, 2-fluorocyclohexylcarbonyl, 2-hydroxycyclopropylcarbonyl, 2-methoxycyclopropylcarbonyl, 2-ethoxycyclopropylcarbonyl and 2-cyanocyclopropylcarbonyl groups are preferred. More preferred groups are the 2-fluorocyclopropylcarbonyl, 2-chlorocyclopropylcarbonyl, 2-fluoracyclobutylcarbonyl and 2-methoxycyclopropyl carbonyl groups, and the most preferred is the 2-fluorocyclopropylcarbonyl group.

In the compounds of the invention according to formula I, where R₂ represents a substituted benzoyl group, this is substituted by at least one of substituents B, which are selected from the group consisting of alkyl groups having from 1 to 4 carbon atoms, halogen atoms and alkoxy groups having from 1 to 4 carbon atoms, all of which may be as exemplified in relation to the same groups and atoms represented by R₁. The number of the substituents may be from 1 to 5, provided that there is no problem of steric hindrance; preferably, however, are from 1 to 3 substituents, more preferably 1 or Specific examples of such substituted benzoyl groups include the 2-fluorobenzoyl, 3-fluorobenzoyl, 4-fluorobenzoyl, 2,4-difluorobenzoyl, 2,4,6-trifluorobenzoyl, 2,3,4,5,6-pentafluorobenzoyl, 4-chlorobenzoyl, 2,4-dichlorobenzoyl, 4-methylbenzoyl, 2,4-dimethylbenzoyl, 4-ethylbenzoyl, 2,4-diethylbenzoyl, 4-methoxybenzoyl, 2,4-dimethoxybenzoyl, 4-ethoxybenzoyl and 2,4-diethoxybenzoyl groups, of which the 4-fluorobenzoyl and 2,4-difluorobenzoyl groups are preferred.

In the compounds of the invention according to formula I, where R₃ represents an alkoxy group, this may be a straight or branched chain group having from 1 to 4 carbon atoms and may be any of the alkoxy groups exemplified above in relation to R₁. Such a group may be unsubstituted or it may have one or more substituents selected from the group consisting of substituents C, defined above, and examples of which are as follows: alkoxy groups having from 1 to 4 carbon atoms, such as those exemplified above in relation to R₁; alkanoyloxy groups having from 1 to 6 carbon atoms, which may be a straight or branched chain group, for example the formyloxy, acetoxy, propionyloxy, butyryloxy, isobutyryloxy, valeryloxy, isovaleryloxy, pivaloyloxy or hexanoyloxy groups, of which those groups having from 1 to 5 carbon atoms are preferred, and the acetoxy, propionyloxy, butyryloxy and pivaloyloxy groups are most preferred; and arylcarbonyloxy groups in which the aryl part is as defined above, for example the arylcarbonyloxy groups exemplified below in relation to R₃. Specific examples of such substituted alkoxy groups include the methoxymethoxy, ethoxymethoxy, propoxymethoxy, butoxymethoxy, 2-methoxyethoxy, 2-ethoxyethoxy, formyloxymethoxy, acetoxymethoxy, propionyloxymethoxy, 2-formyloxyethoxy, 2-acetoxyethoxy, 2-propionyloxyethoxy, 3-acetoxypropoxy, 4-acetoxybutoxy, valeryloxymethoxy, pivaloyloxymethoxy, benzoyloxymethoxy, naphthoyloxymethoxy, p-toluoyloxymethoxy, p-chlorobenzoyloxymethoxy, 2-benzoyloxyethoxy, 3-benzoyloxypropoxy and 4-benzoyloxybutoxy groups, of which the pivaloyloxymethoxy group is most preferred.

In the compounds of the invention according to formula I, where R₃ represents an aralkyloxy group, the alkoxy part is an alkoxy group having from 1 to 4, preferably from 1 to 3, carbon atoms, such as those exemplified above in relation to R₁, especially the methoxy, ethoxy, propoxy or isopropoxy groups. The aryl part is as defined above and has from 6 to 10, preferably 6 or 10, ring carbon atoms. Examples of such aryl groups include the phenyl, 1-naphthyl and 2-naphthyl groups and such groups which are substituted by one or more of substituents D, defined above and examples of which have been given in relation to the same groups and atoms which may be represented by R₁. The alkoxy part may be substituted by one or more aryl groups, the maximum being dictated only by the number of substitutable positions and possibly also by steric constraints; however, from 1 to 3 aryl groups are normally preferred, 1 or 2 being more preferred and 1 being most preferred. Specific examples of the aralkyloxy groups include the benzyloxy, 1-naphthylmethoxy, 2-naphthylmethoxy, phenethyloxy, .alpha.-methylbenzyloxy, 3-phenylpropoxy, 2-phenylpropoxy, 1-phenylpropoxy, 4-phenylbutoxy, benzhydryloxy (i.e. diphenylmethoxy) and trityloxy (i.e. triphenylmethoxy) groups (of these, the benzyloxy and phenethyloxy groups are preferred), and such groups which are substituted by one or more of substituents D.

In the compounds of the invention according to formula I, where R₃ represents an alkanoyloxy group, this may be a straight or branched chain group and has from 1 to 18 carbon atoms. Examples of such groups include the formyloxy, acetoxy, propionyloxy, butyryloxy, isobutyryloxy, valeryloxy, isovaleryloxy, pivaloyloxy, hexanoyloxy, heptanoyloxy, octanoyloxy, nonanoyloxy, decanoyloxy, lauroyloxy, myristoyloxy, palmitoyloxy and stearoyloxy groups, of which those groups having from 1 to 12 carbon atoms are preferred, those having from 2 to 10 carbon atoms are more preferred, and those having from 2 to 5 carbon atoms are most preferred, especially the acetoxy, propionyloxy, butyryloxy, pivaloyloxy, nonanoyloxy and decanoyloxy groups, of which the acetoxy, propionyloxy, butyryloxy and pivaloyloxy groups are most preferred.

In the compounds of the invention according to formula I, where R₃ represents an alkenoyloxy group, this may be a straight or branched chain group and has from 3 to 6, more preferably 3 or 4, carbon atoms. Examples of such groups include the acryloyloxy, methacryloyloxy, 2-butenoyloxy, 2-pentenoyloxy and 2-hexenoyloxy groups, of which the acryloyloxy and methacryloyloxy groups are preferred.

In the compounds of the invention according to formula I, where R₃ represents a cycloalkylcarbonyloxy group, this has from 4 to 8, more preferably from 4 to 7, carbon atoms, that is the cycloalkyl group itself has from 3 to 7 ring carbon atoms. Examples of such groups include the cyclopropylcarbonyloxy, cyclobutylcarbonyloxy, cyclopentylcarbonyloxy, cyclohexylcabonyloxy and cycloheptylcarbonyloxy groups, of which the cyclopropylcarbonyloxy and cyclobutylcarbonyloxy groups are particularly preferred.

In the compounds of the invention according to formula I, where R₃ represents arylcarbonyloxy group, the aryl part is as defined above, and examples of such groups include the benzoyloxy, 1-naphthoyloxy, 2-naphthoyloxy, o-, m- and p-toluoyloxy, o-, m- and p-chlorobenzoyloxy, o-, m- and p-fluorobenzoyloxy, o-, m- and p-methoxybenzoyloxy, 2,3-, 2,4-, 2,5-, 2,6-, 3,4- and 3,5-dichlorobenzoyloxy, 2,4-difluorobenzoyloxy and 2,4,6-trifluorobenzoyloxy groups, preferably the benzoyloxy group.

In the compounds of the invention according to formula I, where R₃ represents an alkoxycarbonyloxy group, this may be a straight or branched chain alkoxycarbonyloxy group having from 2 to 5 carbon atoms, that is the alkoxy part has from 1 to 4 carbon atoms, and examples include the methoxycarbonyloxy, ethoxycarbonyloxy, propoxycarbonyloxy, isopropoxycarbonyloxy, butoxycarbonyloxy, isobutoxycarbonyloxy, sec-butoxycarbonyloxy and t-butoxycarbonyloxy groups. Of these, we prefer those alkoxycarbonyloxy groups having from 1 to 3 carbon atoms in the alkoxy part and the t-butoxycarbonyloxy group, more preferably the methoxycarbonyloxy, ethoxycarbonyloxy and t-butoxycarbonyloxy groups. In the compounds of the invention according to formula I, where R₃ represents an aralkyloxycarbonyloxy group, the alkoxy part is an alkoxy group having from 1 to 4, preferably from 1 to 3, carbon atoms, such as those exemplified above in relation to R₁, especially the methoxy, ethoxy, propoxy or isopropoxy groups. The aryl part is as defined above and has from 6 to 10, preferably 6 or 10, ring carbon atoms. Examples of such aryl groups include the phenyl, 1-naphthyl and 2-naphthyl groups and such groups which are substituted by one or more of substituents D, defined above and examples of which have been given in relation to the same groups and atoms which may be represented by R₁. The alkoxy part may be substituted by one or more aryl groups, the maximum being dictated only by the number of substitutable positions and possibly also by steric constraints; however, from 1 to 3 aryl groups are normally preferred, 1 or 2 being more preferred and 1 being most preferred. Specific examples of the aralkyloxycarbonyloxy groups include the benzyloxycarbonyloxy, 1-naphthylmethoxycarbonyloxy, 2-naphthylmethoxycarbonyloxy, phenethyloxycarbonyloxy, α-methylbenzyloxy carbonyloxy, 3-phenylpropoxycarbonyloxy, 2-phenylpropoxycarbonyloxy, 1-phenylpropoxycarbonyloxy, 4-phenylbutoxycarbonyloxy, benzhydryloxycarbonyloxy and trityloxycarbonyloxy groups (of these, the benzyloxycarbonyloxy group is preferred), and such groups which are substituted by one or more of substituents D.

In the compounds of the invention according to formula I, where R₃ represents a group of formula -NR^{a}R^{b}, R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined above. Examples of the alkyl groups which may be represented by R^{a} and R^{b} are as given above in relation to R₁, and examples of the substituted alkyl groups which may be represented by R^{a} and R^{b} are the substituted alkyl groups corresponding to the substituted alkoxy groups, as given above in relation to R₃. Specific examples of these groups of formula -NR^{a}R^{b} include amino, methylamino, ethylamino, propylamino, isopropylamino, butylamino, isobutyamino, sec-butylamino, t-butylamino, dimethylamino, diethylamino, dipropylamino, diisopropylamino, dibutylamino, methylethylamino, methylpropylamino, N-(methoxymethyl)amino, N-(2-methoxyethyl)amino, N-(acetoxymethyl)amino, N-(pivaloyloxymethyl)amino, N-(benzoylmethyl)amino, N-(2-acetoxyethyl)amino, N-(2-pivaloyloxyethyl)amino and N-(2-benzoylethyl)amino groups, preferably the amino, methylamino, ethylamino, N-(acetoxymethyl)amino and N-(pivaloyloxymethyl)amino groups.

In the compounds of the invention according to formula I, where R₃ represents an aralkylamino group, the alkyl part is an alkyl group having from 1 to 4, preferably from 1 to 3, carbon atoms, such as those exemplified above in relation to R₁, especially the methyl, ethyl, propyl or isopropyl groups. The aryl part is as defined above and has from 6 to 10, preferably 6 or 10, ring carbon atoms. Examples of such aryl groups include the phenyl, 1-naphthyl and 2-naphthyl groups and such groups which are substituted by one or more of substituents D, defined above and examples of which have been given in relation to the same groups and atoms which may be represented by R₁. The alkyl part may be substituted by one or more aryl groups, the maximum being dictated only by the number of substitutable positions and possibly also by steric constraints; however, from 1 to 3 aryl groups are normally preferred, 1 or 2 being more preferred and 1 being most preferred. Specific examples of the aralkyl amino groups include the benzylamino, N-(1-naphthylmethyl)amino, N-(2-naphthylmethyl)amino, phenethylamino, N-(.alpha.-methylbenzyl)amino, N-(3-phenylpropyl) amino, N-(2-phenylpropyl)amino, N-(1-phenylpropyl)amino, N-(4-phenylbutyl)amino, benzhydrylamino and tritylamino groups (of these, the benzylamino group is preferred), and such groups which are substituted by one or more of substituents D.

In the compounds of the invention according to formula I, where R₃ represents an alkanoylamino group, this may be a straight or branched chain group and has from 1 to 18 carbon atoms. Examples of such groups include the formamido, acetamido, propionamido, butyramido, isobutyramido, valerylamino, isovalerylamino, pivaloylamino, hexanoylamino, heptanoylamino, octanoylamino, nonanoylamino, decanoylamino, lauroylamino, myristoylamino, palmitoylamino and stearoylamino groups, of which those groups having from 1 to 12 carbon atoms are preferred, those having from 2 to 10 carbon atoms are more preferred, and those having from 2 to 5 carbon atoms are most preferred, especially the acetamido, propionamido, butyramido, pivaloylamino, nonanoylamino and decanoylamino groups, of which the acetamido, propionamido, butyramido and pivaloylamino groups are most preferred.

In the compounds of the invention according to formula I, where R₃ represents an alkenoylamino group, this may be a straight or branched chain group and has from 3 to 6 carbon atoms. Examples of such groups include the acryloylamino, methacryloylamino, 2-butenoylamino, 2-pentenoylamino and 2-hexenoylamino groups, of which the acryloylamino and methacryloylamino groups are preferred.

In the compounds of the invention according to formula I, where R₃ represents a cycloalkylcarbonylamino group, this has from 4 to 8 carbon atoms, that is the cycloalkyl group itself has from 3 to 7 ring carbon atoms. Examples of such groups include the cyclopropylcarbonylamino, cyclobutylcarbonylamino, cyclopentylcarbonylamino, cyclohexylcarbonylamino and cycloheptylcarbonylamino groups, of which the cyclopropylcarbonylamino and cyclobutylcarbonylamino groups are particularly preferred.

In the compounds of the invention according to formula I, where R₃ represents arylcarbonylamino group, the aryl part is as defined above, and examples of such groups include the benzamido, 1-naphthoylamino, 2-naphthoylamino, o-, m- and p-toluoylamino, o-, m- and p-chlorobenzamido, o-, m- and p-fluorobenzamido, o-, m- and p-methoxybenzamido, 2,4-dichlorobenzamido, 2,4-difluorobenzamido and 2,4,6-trifluorobenzamido groups, preferably the benzamido group.

In the compounds of the invention according to formula I, where R₃ represents an alkoxycarbonylamino group, this may be a straight or branched chain alkoxycarbonylamino group having from 2 to 5 carbon atoms, that is the alkoxy part has from 1 to 4 carbon atoms, add examples include the methoxycarbonylamino, ethoxycarbonylamino, propoxycarbonylamino, isopropoxycarbonylamino, butoxycarbonylamino, isobutoxycarbonylamino, sec-butoxycarbonylamino and t-butoxycarbonylamino groups. Of these, we prefer those alkoxycarbonylamino groups having from 1 to 3 carbon atoms in the alkoxy part and the t-butoxycarbonylamino group, more preferably the methoxycarbonylamino, ethoxycarbonylamino and t-butoxycarbonylamino groups.

In the compounds of the invention according to formula I, where R₃ represents an aralkoxycarbonylamino group, the alkoxy part is an alkoxy group having from 1 to 4, preferably from 1 to 3, carbon atoms, such as those exemplified above in relation to R₁, especially the methoxy, ethoxy, propoxy or isopropoxy groups. The aryl part is as defined above and has from 6 to 10, preferably 6 or 10, ring carbon atoms. Examples of such aryl groups include the phenyl, 1-naphthyl and 2-naphthyl groups and such groups which are substituted by one or more of substituents D, defined above and examples of which have been given in relation to the same groups and atoms which may be represented by R₁. The alkoxy part may be substituted by one or more aryl groups, the maximum being dictated only by the number of substitutable positions and possibly also by steric constraints; however, from 1 to 3 aryl groups are normally preferred, 1 or 2 being more preferred and 1 being most preferred. Specific examples of the aralkyloxycarbonylamino groups include the b enzyloxycarbonyl amino, N-(1-naphthylmethoxycarbonyl)amino, N-(2-naphthylmethoxycarbonyl)amino, phenethyloxycarbonylamino, N-(.alpha.-methylbenzyloxycarbonyl)amino, N-(3-phenylpropoxycarbonyl)amino, N-(2-phenylpropoxycarbonyl)amino, N-(1-phenylpropoxycarbonyl)amino, N-(4-phenylbutoxycarbonyl)amino, benzhydryloxycarbonylamino and trityloxycarbonylamino groups (of these, the benzyloxycarbonylamino group is preferred), and such groups which are substituted by one or more of substituents D.

In the compounds of the invention according to formula I, R₃ may be at either the 2- or the 3-position of the tetrahydrothienopyridyl group, but is preferably at the 2-position.

In the compounds of the present invention, the carbon atom to which the group represented by R₂ is attached is an assymetric carbon atom, and other carbon atoms may be assymetric, and the compounds accordingly form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

In addition, when the compounds of the present invention have one or more carbon-carbon double bonds or one or more disubstituted cycloalkyl moieties, they form cis and trans isomers. The present invention includes both the individual, isolated isomers and mixtures thereof.

The compounds of the present invention can form acid addition salts. There is no particular restriction on the nature of these salts, provided that, where they are intended for therapeutic use, they are pharmaceutically acceptable. Where they are intended for non-therapeutic uses, e.g. as intermediates in the preparation of other, and possibly more active, compounds, even this restriction does not apply. Examples of such acid addition salts include: salts with mineral acids, especially hydrohalic acids (such as hydrofluoric acid, hydrobromic acid, hydroiodic acid or hydrochloric acid), nitric acid, carbonic acid, sulfuric acid or phosphoric acid; salts with lower alkylsulfonic acids, such as methanesulfonic acid, trifluoromethanesulfonic acid or ethanesulfonic acid; salts with arylsulfonic acids, such as benzenesulfonic acid or p-toluenesulfonic acid; and salts with organic carboxylic acids, such as acetic acid, propionic acid, butyric acid, fumaric acid, tartaric acid, oxalic acid, malonic acid, maleic acid, malic acid, succinic acid, benzoic acid, mandelic acid, ascorbic acid, lactic acid, gluconic acid or citric acid.

The compounds of the present invention may also readily form hydrates and these, also, form part of the present invention.

Additionally, when R₃ represents an amino group or a substituted amino group, the resulting compound can form a complex salt with a metal ion, and such complex salts also form part of the present invention. Examples of such complex salts include salts with calcium chloride, magnesium chloride, zinc chloride, ferric chloride, stannic chloride and nickel chloride.

### Concerning formula II:

In the compounds of the invention according to formula II the term "aryl" shall mean a mono- or polycylic ring system composed of 5- and 6- membered aromatic rings containing 0, 1, 2, 3, or 4 heteroatoms chosen from N, O, and S and either unsubstitutued or substituted with R ⁶.

In the compounds of the invention according to formula II the term "alkyl" shall mean straight or branched chain alkane, alkene or alkyne.

In the compounds of the invention according to formula II the terms "aralkyl" and "alkaryl" shall be taken to include an alkyl portion where alkyl is as defined above and to include an aryl portion where aryl is as defined above.

### Concerning formula III:

In the compounds of the invention according to formula III the lower alkyl groups defined in R₉, R₈ and R₁₀ are represented by straight- or branched-chain alkyl groups having 1 to 4 carbon atoms, which may be more definitely specified as methyl, ethyl, propyl, isopropyl, butyl, isobutyl or tert-butyl groups or the like.

In the compounds of the invention according to formula III the lower alkenyl group defined in R₉ is represented by a straight- or branched-chain alkenyl group having 2 to 4 carbon atoms, which may be more definitely specified as vinyl, allyl, crotyl or 1-methylallyl group or the like.

In the compounds of the invention according to formula III the lower alkanoyl group defined in R₉ is represented by a straight- or branched-chain alkanoyl group having 1 to 4 carbon atoms, which may be definitely specified as formyl, acetyl, propionyl, butyryl or isobutyryl group or the like.

In the compounds of the invention according to formula III the phenylalkyl group defined in R₉ and R₁₀ is represented by a phenylalkyl group in which the straight- or branched-chain alkyl group having 1 to 4 carbon atoms, having one or more phenyl group(s), which may be definitely specified as benzyl, α-phenethyl, β-phenethyl, 3-phenylpropyl, 4-phenylbutyl, 1,1-dimethyl-2-phenylethyl, diphenylmethyl group or the like.

In the compounds of the invention according to formula III the cycloalkyl group defined in R₁₀ is represented by a cycloalkyl group having 3 to 8 carbon atoms, which may be definitely specified as cyclopropyl, cyclobutyl, cyclopentyl, cyclohexyl, cycloheptyl or cyclooctyl group or the like.

In the compounds of the invention according to formula III the cycloalkylalkyl group defined in R₁₀ is represented by a cycloalkylalkyl group in which the straight- or branched-chain alkyl group having 1 to 4 carbon atoms, having one or more cycloalkyl group(s), which may be definitely specified as cyclopropylmethyl, cyclobutylmethyl, cyclopentylmethyl, cyclohexylmethyl, cyclooctylmethyl, dicyclohexylmethyl, 2-cyclohexylethyl, 2-cyclooctylethyl, 1-methyl-2-cyclohexylethyl, 3-cycloheptylpropyl, 4-cyclohexylbutyl, 1,1-dimethyl-2-cyclohexylethyl group or the like.

As to the substituted groups on the phenyl group in the said benzoyl group, phenylalkyl group and phenyl group, they may be definitely specified as lower alkoxy group(s) for example, methoxy, ethoxy, propoxy, isopropoxy, butoxy or tert-butoxy group(s); lower alkyl group(s) for example, methyl, ethyl, propyl, isopropyl, butyl, isobutyl or tert-butyl group(s); halogen atom(s) for example, chlorine, fluorine, bromine atom(s); di-lower alkylamino group(s) for example, dimethylamino, diethylamino, dipropylamino, dibutylamino or methylethylamino group(s); nitro group(s); lower alkylenedioxy group(s) for example, methylenedioxy, ethylenedioxy or trimethylenedioxy group(s).

The phenyl, phenylalkyl or benzoyl group having said substituted group(s) may be exemplified as a substituted phenyl group for example, 4-chlorophenyl, 3,5-dichlorophenyl, 2-bromophenyl, 4-methoxyphenyl, 3,4-dimethoxyphenyl, 2-nitrophenyl, 3,4,5-trimethoxyphenyl, 2-methylphenyl, 4-ethylphenyl, 3,4-dimethylphenyl, 3,4-methylenedioxyphenyl, 2-chloro-4-nitrophenyl or 4-dimethylaminophenyl group; a substituted benzoyl group for example, 4-chlorobenzoyl, 3,5-dichlorobenzoyl, 2-bromobenzoyl, 4-methoxybenzoyl, 3,4-dimethoxybenzoyl, 2-nitrobenzoyl, 3,4,5-trimethoxybenzoyl, 2-methylbenzoyl, 4-ethylbenzoyl, 3,4-dimethylbenzoyl, 3,4-methylenedioxybenzoyl, 2-chloro-4-nitrobenzoyl or 4-dimethylaminobenzoyl group; a substituted phenylalkyl group for example, 4-chlorobenzyl, 3,5-dichlorobenzyl, β-2-bromophenethyl, 4-methoxybenzyl, β-3,4-dimethoxyphenethyl, 2-nitrobenzyl, β-3,4,5-trimethoxyphenethyl, 2-methylbenzyl, α-4-ethylphenethyl, β-3,4-dimethylphenethyl, β-3,4-methylenedioxyphenethyl, 2-chloro-4-nitrobenzyl or β-4-dimethylaminophenethyl group.

Furthermore, the lower alkylene group defined as A in the compounds of the invention according to formula III is represented by a straight- or branched-chain alkylene group having 1 to 6 carbon atoms, which may be more definitely specified as methylene, ethylene, trimethylene, propylene, tetramethylene, 2-ethylethylene, pentamethylene, hexamethylene, 2-methyltrimethylene, 2,2-dimethyltrimethylene, or 1-methyltrimethylene group.

### Concerning formula IV:

In the compounds of the invention according to formula IV all alkyl groups when present as such, or as a moiety in other groups such as alkoxy, will be lower alkyl groups composed of 1-6 carbon atoms, preferably 1-5 carbon atoms, and more usually 1-4 carbon atoms with C₁, C₂ and C₃ often being of particular interest.

### Concerning formula V:

In the compounds of the invention according to formula V alkyl groups, whether alone or as part of another group, can be straight chained or branched.

### EXAMPLES:

### Example 1:

| | |
|---|---|
| Personal Description: | 8 year old Shetty-gelding |
| | Weight: ca. 250 kg |
| | Charcteristic: as in registration certificate |
| | Dental notation: according to declared age |
| | The gelding is standing in its box and is daily on the pasture |
| | State of vaccination / Deworming: routine |
| Task: | Treatment of *Pododermatitis aseptica chronica,* acute attack Since several years always recurring laminitis attacks, miscellaneous treatments of veterinarians (bloodletting, NSAID (non steroidal anti inflammatory drugs), cortisone) always recurring hoof ulcers |
| First examination (day 1): | The hind legs are below the body and the hoofs cannot be lifted. |
| | The horse shows high-grade supporting leg lameness. The horse is not horseshoed. |
| | Feeding and maintenance conditions are very good. The horse is apathetic and suffers a lot of pain. The episcleral vessels are washed out. |
| Treatment: | an initial dosage of 20 mg Prasugrel in combination with 1000 mg acetylsalicylic acid per 60 kg, 10 mg Prasugrel in combination with 500 mg acetylsalicylic acid per 60 kg twice a day and change in feeding |
| Therapy progress: | day 2: distinct pulsation in all 4 legs; the horse is more interested in its surrounding; 24 h later (day 3) pulsation in the front legs less, in the back legs the same; horse comes when it is called. |
| 1. Follow-up examination: | day 4: pulsation in all 4 legs; horse walks with medium grade turning pain. |
| 2. Follow-up examination: | day 6: no signs of laminitis. On day 8 no hoof ulcers observable. On day 10 stop of treatment, further keeping change of feeding. Since then no more laminitis occurred. |

### Example 2:

| | |
|---|---|
| Personal Description: | 8 year old Shetty-mare |
| | Weight: ca. 200 kg |
| | Charcteristic: as in registration certificate |
| | Dental notation: according to declared age |
| | At night in its box and during the day on the pasture |
| | State of vaccination: Deworming unclear |
| Task: | Treatment of *Pododermatitis aseptica* |
| | Heavily overweight, fat deposition in the neck area, always recurring laminitis attacks since a couple of years, miscellaneous treatments of veterinarians (bloodletting, NSAID, cortisone) always recurring hoof ulcers |
| First examination (day 1): | Lameness on all 4 legs. Very pressure sensitive on the sole. |
| Treatment: | per 60 kg 10 mg Prasugrel in combination with 500 mg acetylsalicylic acid twice a day and change in feeding |
| Therapy progress: | light amelioration from day 3 on; pulsation clearly sensible until day 5; no pulsation anymore sensible from day 7 on; stop of treatment on day 14. |
| Follow-up examination: | 1 month later: horse gained a lot of weight due to pregnancy; no pulsation; attitude without pathological findings; 8 days later: normal birth of a healthy foal |

### Example 3:

| | |
|---|---|
| Personal Description: | 12 year old Pony-gelding |
| | Weight: ca. 300 kg |
| | Charcteristic: as in registration certificate |
| | Dental notation: according to declared age |
| | At night in its box and during the day on the pasture and the outdoor riding facility |
| | State of vaccination: Deworming unclear |
| | Usage: riding pony |
| Task: | Treatment of *Pododermatitis aseptica chronica* |
| | Always recurring laminitis attacks since a couple of years, miscellaneous unsuccessful treatments of veterinarians |
| First examination (day 1): | Lameness on all 4 legs; pressure sensitive; no turn possible anymore. |
| Treatment: | 10 mg Prasugrel in combination with 500 mg acetylsalicylic acid per 60 kg twice a day and change in feeding |
| Therapy progress: | light amelioration from day 2 on; no pulsation anymore sensible from day 4 on; day 6: pony walks still restrained; day 10: unlimited movement; stop of treatment on day 12. |

### Example 4:

| | |
|---|---|
| Personal Description: | 8 year old Shetty-gelding |
| | Weight: ca. 200 kg |
| | Charcteristic: as in registration certificate |
| | Dental notation: according to declared age |
| | Lives in an open barn |
| | State of vaccination / Deworming: unclear |
| Task: | Treatment of *Pododermatitis aseptica chronica* |
| | Pony laid mostly in barn; second laminitis attack, first laminitis attack 5 months ago; successful conventional treatments of veterinarians (bloodletting, NSAID) |
| First examination (day 1): | Pony heavily overweight; acute attack with heavy pain; lameness on all 4 legs; |
| Treatment: | 10 mg Prasugrel in combination with 500 mg acetylsalicylic acid per 60 kg twice a day and change in feeding |
| Therapy progress: | day 3: pony standing in bran, but still heavy pain on all 4 legs; standing up until its ankles in the hay; change of surrounding; day 5: clear amelioration of its attitude; less pulsation; from day 10 on no pulsation sensible anymore; stop of treatment on day 14. |

## Claims

1. A platelet aggregation inhibitor for the use in the treatment and/or prevention of laminitis in Ungulates.

2. A compound according to claim 1 or or a tautomer thereof, or a pharmaceutically acceptable salt thereof selected from the group comprising 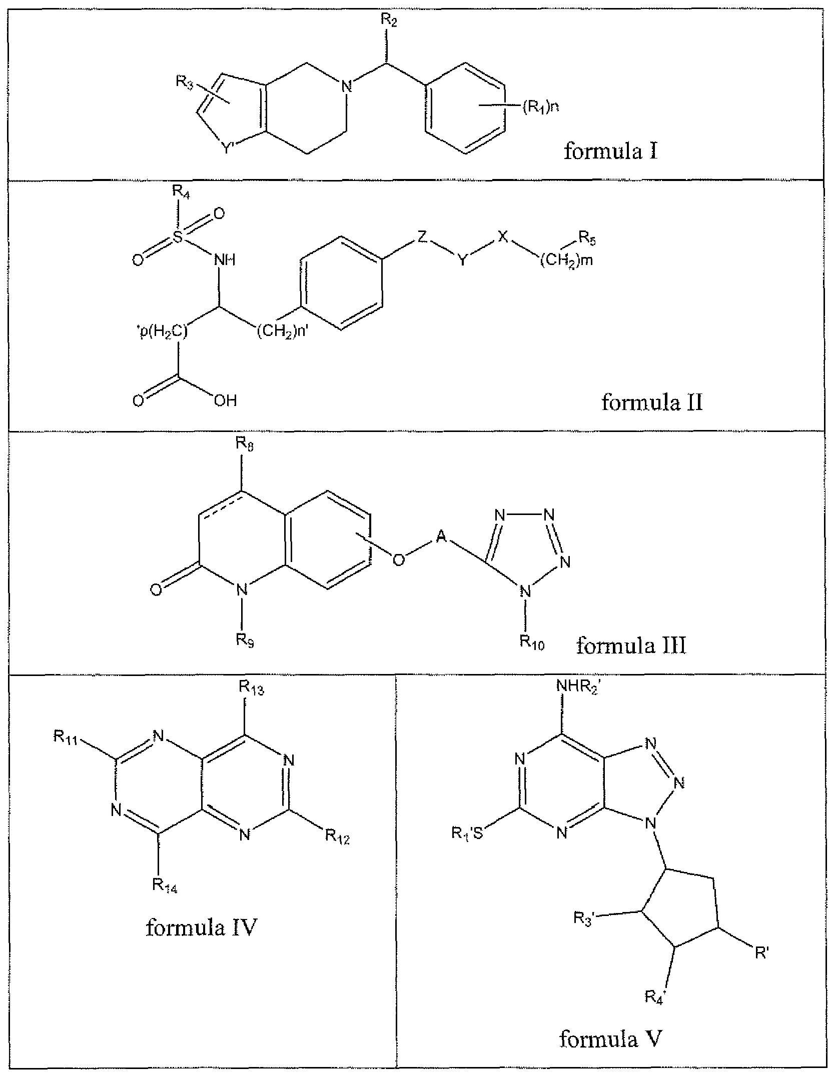 wherein
in formula I
Y' is NH, O or S;
R₁ represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl group;
R₂ represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
R₃ represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxolen-4yl)methoxy group; a (5-phenyl-2-oxo-1,3-dioxolen-4-yl)methoxy group; a group of formula -NR^{a} R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
n is an integer from 1 to 5, and, when n is an integer from 2 to 5, the groups represented by R₁ may be the same as or different from each other;
said substituents A are selected from the group consisting of halogen atoms, hydroxy groups, alkoxy groups having from 1 to 4 carbon atoms and cyano groups;
said substituents B are selected from the group consisting of alkyl groups having from 1 to 4 carbon atoms, halogen atoms and alkoxy groups having from 1 to 4 carbon atoms;
said substituents C are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom;
in formula II
R₄ is aryl, C₁₋₁₀ alkyl or C₄₋₁₀ aralkyl,
Z is a bond or O, -NHC(O)-, -C(O)NH-, C₁₋₅ straight or branched alkyl,
m is an integer from 0 to 6
R₅ is
a) a six membered saturated heterocyclic ring containing one or two heteroatoms wherein said heteroatoms are N or O and wherein said heterocyclic ring is optionally substituted by C₁₋₃ alkyl,
or
d) NR₆R₇, wherein R₆ and R₇ are independently hydrogen or C₁₋₁₀ alkyl
X and Y are independently C₁₋₁₀ alkyl or cycloalkyl, -NHC(O)- or -C(O)NH-
n' is one or two
p' is zero or one
in formula III
R₈ is a hydrogen atom, a lower alkyl group or a group of the formula 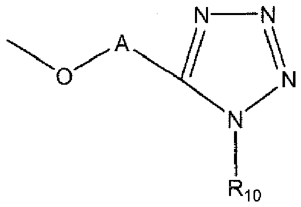
R₉ is a hydrogen atom, a lower alkyl group, a lower alkenyl group, a lower alkanoyl group, a benzoyl group or a phenyl-C.sub.1-4 alkyl group;
R₁₀ is a lower alkyl group, a C₃₋₈ cycloalkyl group, a C₃₋₈ cycloalkyl-C₁₋₄ alkyl group, a phenyl group or a phenyl-C₁₋₄ alkyl group;
A is a lower alkylene group,
the carbon-carbon bond between 3- and 4-positions in the carbostyril skeleton is either single or double bond; and the substituted position of the group represented by the formula, 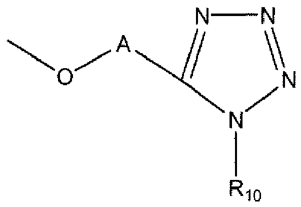 in the carbostyril skeleton is either 4-, 5-, 6-, 7- or 8-position provided that only one such group of the formula can be substituted in the whole carbostyril skeleton, thus when R⁸ in 4-position is 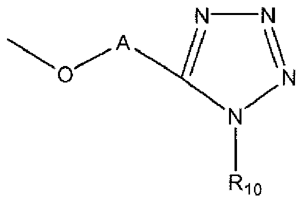 then 5-, 6-, 7- or 8-position will have no such substituted group; furthermore, the phenyl group in the above-mentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy in formula IV
R₁₁ is chloro and R₁₂ is diethanolamino, or
R₁₁/R₁₂ are identical and are selected from allyl, halo, diethanolamino, solketalo and a group having the formula -O-R_{z} or -NHR_{Z},
R_{Z} is selected from alkyl, hydroxyalkyl, alkoxyalkyl, dialkoxyalkyl and 2-oxo-alkyl wherein the or each alkyl and/or alkoxy moiety has less than six carbon atoms, and
R₁₃/R₁₄ are identical and are selected from piperidino, N-tetrahydroisoquinolyl, and a benzylamino group having the following structural formula: 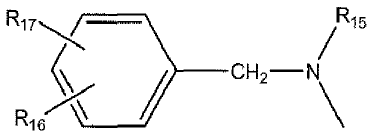 where
R₁₅ is H, or an optionally substituted alkyl or benzyl group, and
R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
R₁' is a C₁₋₆ alkyl, C₂₋₆ alkenyl, C₂₋₆ alkenyl, C₃₋₈-cycloalkyl or a phenyl group, each group being optionally substituted by one or more substituents selected from halogen, OR₈', NR₉'R₁₀', SR₁₁' or C₁₋₆ alkyl (itself optionally substituted by one or more halogen atoms);
R₂' is C₁₋₈ alkyl optionally substituted by one or more substituents selected from halogen, OR₈', NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂ C(O)R₈', OR₈', SR₁₁', NR₁₂' R₁₃', a fused 5- or 6- membered saturated ring containing one or two oxygen atoms, phenyl or C₁₋₆-alkyl the latter two groups being optionally substituted by OR₈', NR₉'R₁₀' or one or more halogen atoms;
R₃'/R₄' one of them is hydroxy and the other is hydrogen, hydroxy or NR₉'R₁₀';
R' is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅'R₆')ⱼR₁₄' where R₅' and R₆' are as defined above, j is 1 to 3 and R₁₄' is COOH, OR₁₅', NR₁₆'R₁₇' or CONR₁₆'R₁₇'; or R' is a C₁₋₄ alkyl or C₂₋₄ alkenyl group, each or which is substituted by one or more groups selected from =S, =O, =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃' are independently hydrogen, C₁₋₄ alkyl or phenyl; R₂₀' is OR₂₄' or NR₂₅'R₂₆', where R₂₄' is hydrogen, C₁₋₄ alkyl or phenyl, and R₂₅' and R₂₆' are independently hydrogen, C₁₋₄ alkyl, aryl, C₁₋₆ acyl, arylsulphonyl or arylcarbonyl);
R₈' is hydrogen, C₁₋₆ alkyl optionally substituted by halogen or R₈' is phenyl optionally substituted by one or more substituents selected from halogen, NO₂, C(O)R₆', OR₆', SR₉', NR₁₀'R₁₁';
R₉'/R₁₀'^{/}R₁₁' are independently hydrogen or C₁₋₆ alkyl;
R₁₂'^{/}R₁₃' are independently hydrogen, C₁₋₆ alkyl, acyl, alkyl sulfonyl optionally substituted by halogen, or phenyl sulfonyl optionally substituted by C₁-C₄ alkyl; and
R₁₅'/R₁₆'/R₁₇' are independently hydrogen C₁₋₆ alkyl;
for the use in the treatment and/or prevention of Laminitis in Ungulates.

3. A compound according to claim 1 and 2 or a tautomer thereof, or a pharmaceutically acceptable salt thereof selected from the group comprising 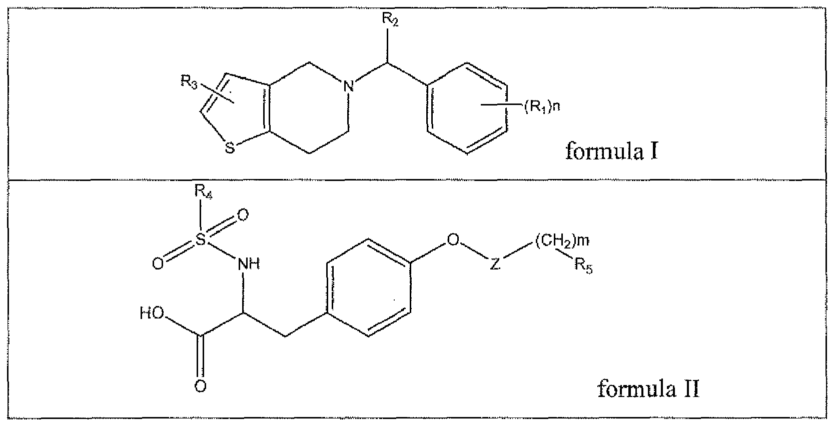 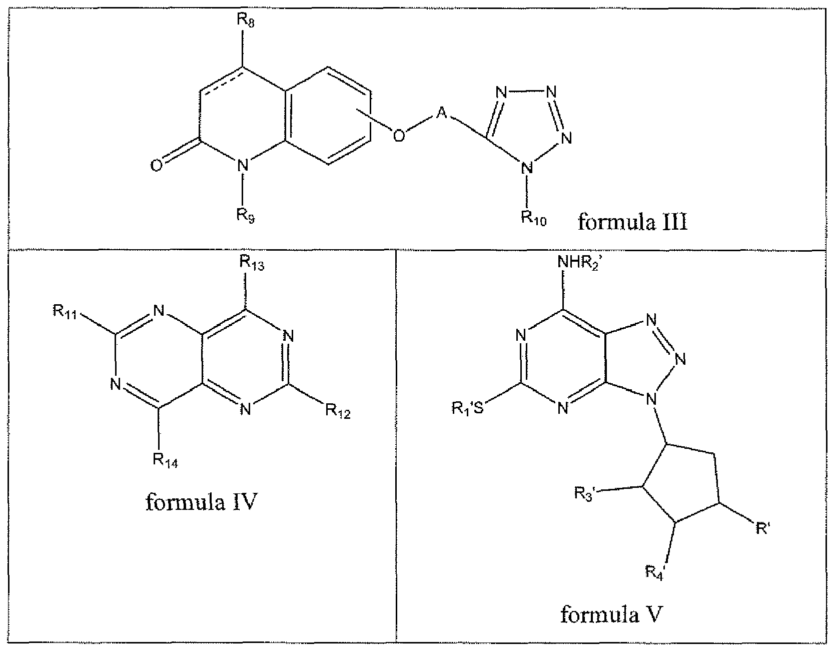 wherein
in formula I
R₁ represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl1 group;
R₂ represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
R₃ represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxolen-4yl)methoxy group; a (5-phenyl-2-oxo-1,3-dioxolen-4-yl)methoxy group; a group of formula -NR^{a} R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
n is an integer from 1 to 5, and, when n is an integer from 2 to 5, the groups represented by R₁ may be the same as or different from each other;
said substituents A are selected from the group consisting of halogen atoms, hydroxy groups, alkoxy groups having from 1 to 4 carbon atoms and cyano groups;
said substituents B are selected from the group consisting of alkyl groups having from 1 to 4 carbon atoms, halogen atoms and alkoxy groups having from 1 to 4 carbon atoms;
said substituents C are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom.
in formula II
R₄ is aryl, C₁₋₁₀ alkyl or C₄₋₁₀ aralkyl,
Z is a bond or O, -NHC(O)-, -C(O)NH-, C₁₋₅ straight or branched alkyl,
m is an integer from 2 to 6
R₅ is
a) a six membered saturated heterocyclic ring containing one or two heteroatoms wherein said heteroatoms are N or O and wherein said heterocyclic ring is optionally substituted by C₁₋₃ alkyl,
or
b) NR₆R₇, wherein R₆ and R₇ are independently hydrogen or C₁₋₁₀ alkyl
in formula III
R₈ is a hydrogen atom, a lower alkyl group or a group of the formula 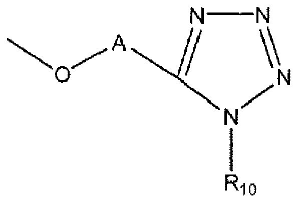
R₉ is a hydrogen atom, a lower alkyl group, a lower alkenyl group, a lower alkanoyl group, a benzoyl group or a phenyl-C.sub.1-4 alkyl group;
R₁₀ is a lower alkyl group, a C₃₋₈ cycloalkyl group, a C₃₋₈ cycloalkyl-C₁₋₄ alkyl group, a phenyl group or a phenyl-C₁₋₄ alkyl group;
A is a lower alkylene group,
the carbon-carbon bond between 3- and 4-positions in the carbostyril skeleton is either single or double bond; and the substituted position of the group represented by the formula, 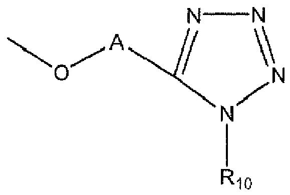 in the carbostyril skeleton is either 4-, 5-, 6-, 7- or 8-position provided that only one such group of the formula can be substituted in the whole carbostyril skeleton, thus when R⁸ in 4-position is 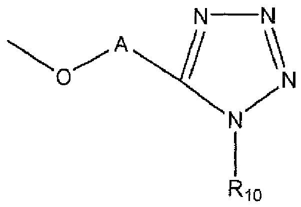 then 5-, 6-, 7- or 8-position will have no such substituted group; furthermore, the phenyl group in the above-mentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy. in formula IV
R₁₁ is chloro and R₁₂ is diethanolamino, or
R₁₁/R₁₂ are identical and are selected from allyl, halo, diethanolamino, solketalo and a group having the formula -O-R_{z} or -NHR_{Z},
R_{z} is selected from alkyl, hydroxyalkyl, alkoxyalkyl, dialkoxyalkyl and 2-oxo-alkyl wherein the or each alkyl and/or alkoxy moiety has less than six carbon atoms, and
R₁₃/R₁₄ are identical and are selected from piperidino, N-tetrahydroisoquinolyl, and a benzylamino group having the following structural formula: 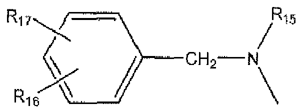 where
R₁₅ is H, or an optionally substituted alkyl or benzyl group, and
R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
R₁' is a C₁₋₆ alkyl, C₂₋₆ alkenyl, C₂₋₆ alkenyl, C₃₋₈-cycloalkyl or a phenyl group, each group being optionally substituted by one or more substituents selected from halogen, OR₈', NR₉'R₁₀', SR₁₁' or C₁₋₆ alkyl (itself optionally substituted by one or more halogen atoms);
R₂' is C₁₋₈ alkyl optionally substituted by one or more substituents selected from halogen, OR₈', NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂, C(O)R₈', OR₈', SR₁₁', NR₁₂'R₁₃', a fused 5- or 6- membered saturated ring containing one or two oxygen atoms, phenyl, or C₁₋₆-alkyl the latter two groups being optionally substituted by OR₈', NR₉'R₁₀' or one or more halogen atoms;
R₃'/R₄' one of them is hydroxy and the other is hydrogen, hydroxy or NR₉'R₁₀';
R' is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅'R₆')ⱼR₁₄' where R₅' and R₆' are as defined above, j is 1 to 3 and R₁₄' is COOH, OR₁₅', NR₁₆'R₁₇' or CONR₁₆'R₁₇'; or R' is a C₁₋₄ alkyl or C₂₋₄ alkenyl group, each or which is substituted by one or more groups selected from =S, =O, =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃' are independently hydrogen, C₁₋₄ alkyl or phenyl; R₂₀' is OR₂₄' or NR₂₅'R₂₆', where R₂₄' is hydrogen, C₁₋₄ alkyl or phenyl, and R₂₅' and R₂₆' are independently hydrogen, C₁₋₄ alkyl, aryl, C₁₋₆ acyl, arylsulphonyl or arylcarbonyl);
R₈' is hydrogen, C₁₋₆ alkyl optionally substituted by halogen or R₈' is phenyl optionally substituted by one or more substituents selected from halogen, NO₂, C(O)R₆', OR₆', SR₉', NR₁₀'R₁₁';
R₉'/R₁₀'^{/}R₁₁' are independently hydrogen or C₁₋₆ alkyl;
R₁₂'^{/}R₁₃' are independently hydrogen, C₁₋₆ alkyl, acyl, alkyl sulfonyl optionally substituted by halogen, or phenyl sulfonyl optionally substituted by C₁-C₄ alkyl; and
R₁₅'/R₁₆'/R₁₇' are independently hydrogen C₁₋₆ alkyl;
for the use in the treatment and/or prevention of Laminitis in Ungulates.

4. A compound or a tautomer thereof, or a pharmaceutically acceptable salt thereof, selected from the group comprising 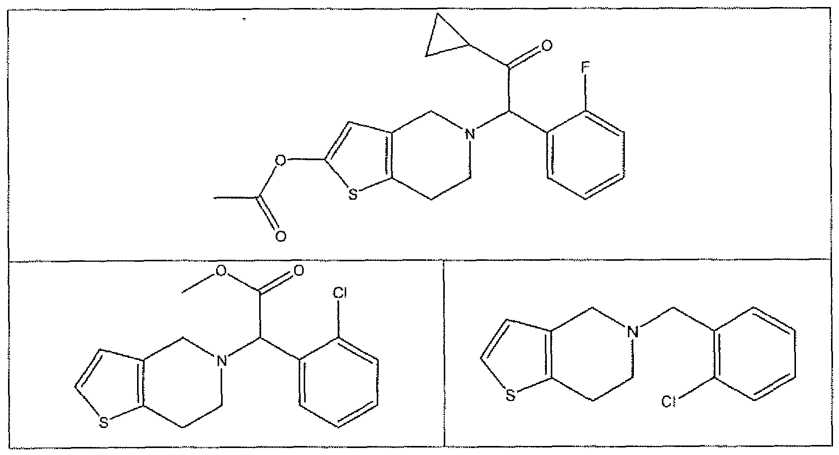 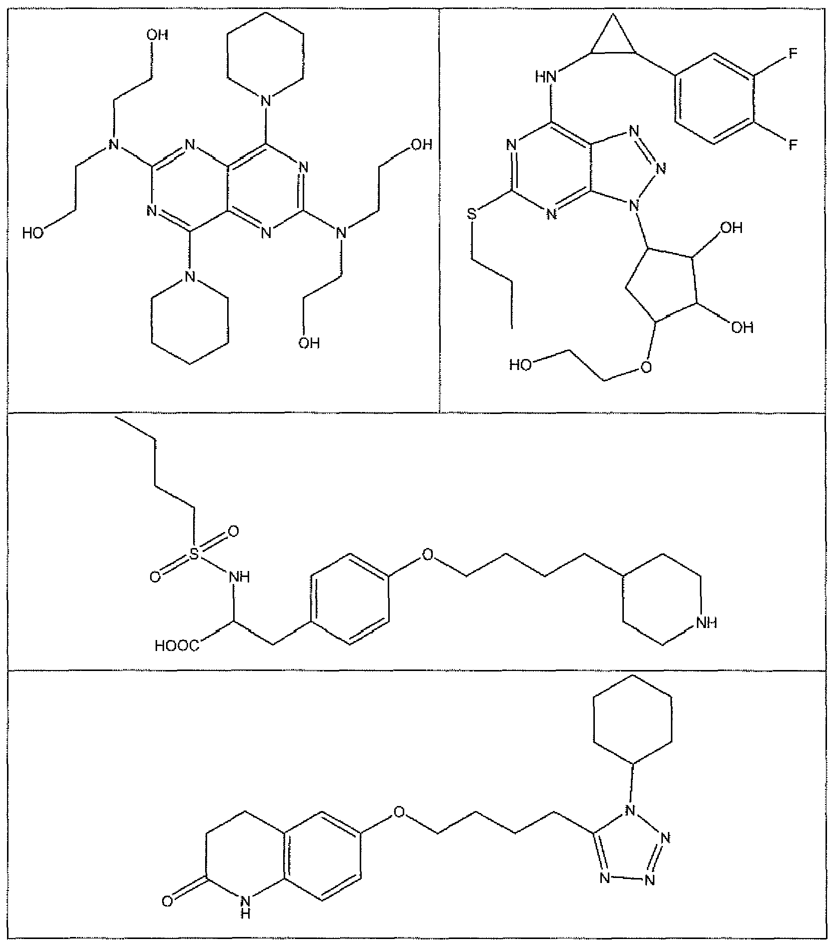 for the use in the treatment and/or prevention of Laminitis in Ungulates.

5. A compound or a tautomer thereof, or a pharmaceutically acceptable salt with the following structure 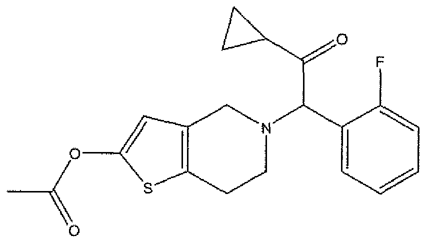 for the use in the treatment of Laminitis in Ungulates.

6. A compound or a tautomer thereof, or a pharmaceutically acceptable salt with the following structure 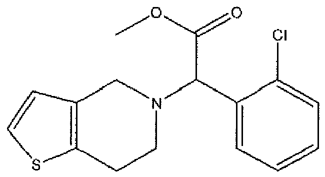 for the use in the treatment and/or prevention of Laminitis in Ungulates.

7. A compound or a tautomer thereof, or a pharmaceutically acceptable salt with the following structure 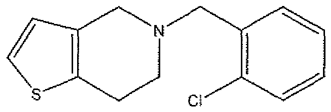 for the use in the treatment and/or prevention of Laminitis in Ungulates.

8. A compound or a tautomer thereof, or a pharmaceutically acceptable salt with the following structure 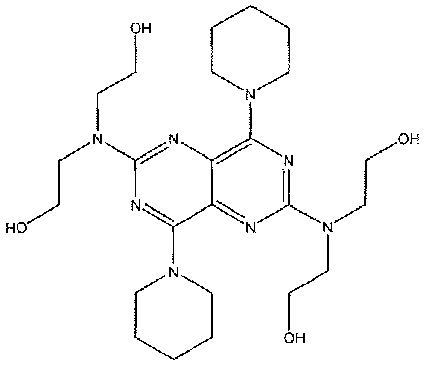 for the use in the treatment and/or prevention of Laminitis in Ungulates.

9. A compound or a tautomer thereof, or a pharmaceutically acceptable salt with the following structure 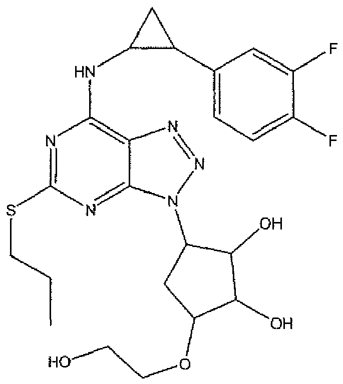 for the use in the treatment and/or prevention af Laminitis in Ungulates.

10. A compound or a tautomer thereof, or a pharmaceutically acceptable salt with the following structure 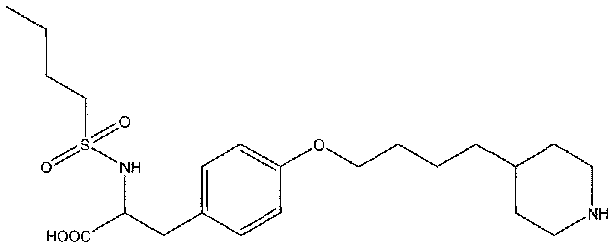 for the use in the treatment and/or prevention of Laminitis in Ungulates.

11. A compound or a tautomer thereof, or a pharmaceutically acceptable salt with the following structure 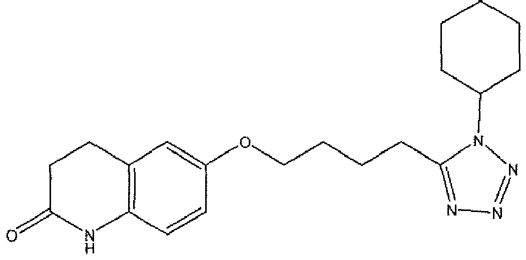 for the use in the treatment and/or prevention of Laminitis in Ungulates.

12. A compound or a tautomer thereof, or a pharmaceutically acceptable salt thereof according to any of the preceding claims in combination with acetylsalicylic acid for the use in the treatment and/or prevention of Laminitis in Ungulates.

13. A compound or a tautomer thereof, or a pharmaceutically acceptable salt with the following structure 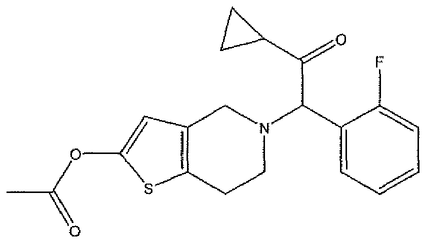 in the dosage of 10 mg in combination with 500 mg acetylsalicylic acid per 60 kg given twice a day for the use in the treatment and/or prevention of Laminitis in Ungulates.

14. A compound of formulae I - V or a tautomer thereof, or a pharmaceutically acceptable salt for the use in the treatment and/or prevention of laminitis in even-toed and odd-toed Ungulates, preferred for bovinae, especially preferred for pregnant bovinae.

15. Animal feed comprising a compound or a tautomer thereof, or a pharmaceutically acceptable salt thereof selected from the group comprising 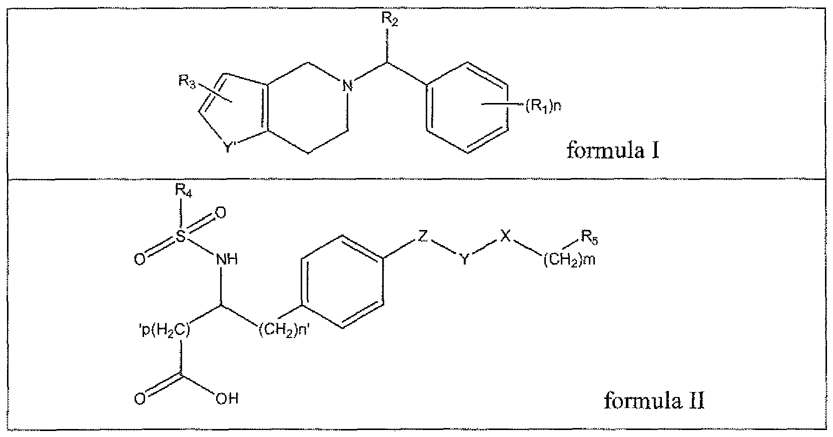 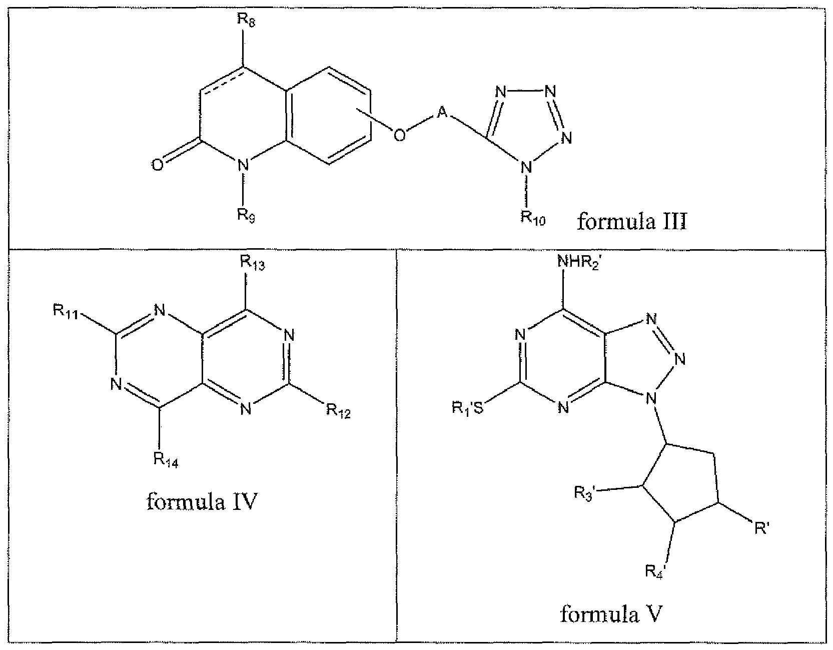 wherein
in formula I
Y' is NH, O or S;
R₁ represents a hydrogen atom, an alkyl group having from 1 to 4 carbon atoms, a halogen atom, a haloalkyl group having from 1 to 4 carbon atoms and at least one halogen atom, a hydroxy group, an alkoxy group having from 1 to 4 carbon atoms, a haloalkoxy group having from 1 to 4 carbon atoms and at least one halogen atom, an alkylthio group having from 1 to 4 carbon atoms, a haloalkylthio group having from 1 to 4 carbon atoms and at least one halogen atom, an amino group, an alkanoyl group having from 1 to 5 carbon atoms, a haloalkanoyl group having from 2 to 5 carbon atoms and at least one halogen atom, a carboxy group, an alkoxycarbonyl group having from 2 to 5 carbon atoms, a carbamoyl group, a cyano group, a nitro group, an alkanesulfonyl group having from 1 to 4 carbon atoms, a haloalkanesulfonyl group having from 1 to 4 carbon atoms and at least one halogen atom, or a sulfamoyl group;
R₂ represents hydrogen or an alkanoyl group having from 1 to 10 carbon atoms; a substituted alkanoyl group which has from 2 to 10 carbon atoms and which is substituted by at least one substituent selected from the group consisting of substituents A, defined below; an alkenoyl group having from 3 to 6 carbon atoms; a substituted alkenoyl group which has from 3 to 6 carbon atoms and which is substituted by at least one substituent selected from the group consisting of substituents A, defined below; a carboxyl group; an alkanoylcarbonyl group having from 2 to 10 carbon atoms; an acetate group; an amide group, wherein the N-atom is substituted twice independently the same or different by hydrogen or a branched alkyl group or the N-atom is part of a heterocycle, which may have one more heteroatom, selected from the group of oxygene or nitrogene and which may be substituted by an alkanoyl group having from 1 to 10 carbon atoms or a benzyl group; a cycloalkylcarbonyl group having from 4 to 8 carbon atoms; a substituted cycloalkylcarbonyl group which has from 4 to 8 carbon atoms and which is substituted by at least one substituent selected from the group consisting of substituents A, defined below; or a substituted benzoyl group having at least one substituent selected from the group consisting of substituents B, defined below;
R₃ represents a hydrogen atom; a hydroxy group; an alkoxy group having from 1 to 4 carbon atoms; a substituted alkoxy group which has from 1 to 4 carbon atoms and which is substituted by at least one substituent selected from the group consisting of substituents C, defined below; an aralkyloxy group in which the aralkyl part is as defined below; an alkanoyloxy group having from 1 to 18 carbon atoms; an alkenoyloxy group having from 3 to 6 carbon atoms; a cycloalkylcarbonyloxy group having from 4 to 8 carbon atoms; an arylcarbonyloxy group in which the aryl part is as defined below; an alkoxycarbonyloxy group having from 2 to 5 carbon atoms; an aralkyloxycarbonyloxy group in which the aralkyl part is as defined below; a phthalidyloxy group; a (5-methyl-2-oxo-1,3-dioxalen-4yl)methoxy group; a (5-phenyl-2-oxo-1,3-dioxolen-4-yl)methoxy group; a group of formula -NR^{a}R^{b} : wherein R^{a} and R^{b} are independently selected from the group consisting of hydrogen atoms, alkyl groups having from 1 to 4 carbon atoms and substituted alkyl groups which have from 1 to 4 carbon atoms and which are substituted by at least one substituent selected from the group consisting of substituents C, defined below; an aralkylamino group in which the aralkyl part is as defined below; an alkanoylamino group having from 1 to 18 carbon atoms; an alkenoylamino group having from 3 to 6 carbon atoms; a cycloalkylcarbonylamino group having from 4 to 8 carbon atoms: an arylcarbonylamino group in which the aryl part is as defined below; an alkoxycarbonylamino group having from 2 to 5 carbon atoms; an aralkyloxycarbonylamino group in which the aralkyl part is as defined below; a phthalidylamino group; a (5-methyl-2-oxo-1,3-dioxolen-4-yl)methylamino group; a (5-phenyl-2-oxo-1,3-dioxolen-4-yl)methylamino group, or a nitro group;
n is an integer from 1 to 5, and, when n is an integer from 2 to 5, the groups represented by R₁ may be the same as or different from each other;
said substituents A are selected from the group consisting of halogen atoms, hydroxy groups, alkoxy groups having from 1 to 4 carbon atoms and cyano groups;
said substituents B are selected from the group consisting of alkyl groups having from 1 to 4 carbon atoms, halogen atoms and alkoxy groups having from 1 to 4 carbon atoms;
said substituents C are selected from the group consisting of alkoxy groups having from 1 to 4 carbon atoms, alkanoyloxy groups having from 1 to 6 carbon atoms and arylcarbonyloxy groups in which the aryl part is as defined below;
said aralkyl parts of said aralkyloxy, aralkyloxycarbonyloxy, aralkylamino and aralkyloxycarbonylamino groups are alkyl groups which have from 1 to 4 carbon atoms and which are substituted by at least one aryl groups as defined below;
said aryl groups and said aryl parts of said arylcarbonyloxy groups and of said arylcarbonylamino groups having from 6 to 10 carbon atoms in a carbocyclic ring which is unsubstituted or is substituted by at least one substituent selected from the group consisting of substituents D, defined below; and said substituents D are selected from the group consisting of the groups and atoms defined above in relation to R₁, other than said hydrogen atom;
in formula II
R₄ is aryl, C₁₋₁₀ alkyl or C₄₋₁₀ aralkyl,
Z is a bond or O, -NHC(O)-, -C(O)NH-, C₁₋₅ straight or branched alkyl,
m is an integer from 0 to 6
R₅ is
a) a six membered saturated heterocyclic ring containing one or two heteroatoms wherein said heteroatoms are N or O and wherein said heterocyclic ring is optionally substituted by C₁₋₃ alkyl, or
e) NR₆R₇, wherein R₆ and R₇ are independently hydrogen or C₁₋₁₀ alkyl
X and Y are independently C₁₋₁₀ alkyl or cycloalkyl, -NHC(O)- or -C(O)NH-
n' is one or two
p' is zero or one
in formula III
R₈ is a hydrogen atom, a lower alkyl group or a group of the formula 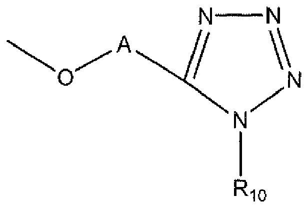
R₉ is a hydrogen atom, a lower alkyl group, a lower alkenyl group, a lower alkenoyl group, a benzoyl group or a phenyl-C.sub.1-4 alkyl group;
R₁₀ is a lower alkyl group, a C₃₋₈ cycloalkyl group, a C₃₋₈ cycloalkyl-C₁₋₄ alkyl group, a phenyl group or a phenyl-C₁₋₄ alkyl group;
A is a lower alkylene group,
the carbon-carbon bond between 3- and 4-positions in the carbostyril skeleton is either single or double bond; and the substituted position of the group represented by the formula, 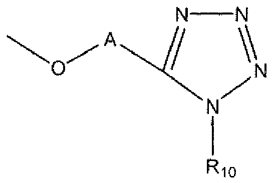 in the carbostyril skeleton is either 4-, 5-, 6-, 7- or 8-position provided that only one such group of the formula can be substituted in the whole carbostyril skeleton, thus when R⁸ in 4-position is 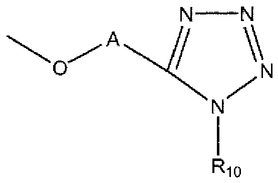 then 5-, 6-, 7- or 8-position will have no such substituted group; furthermore, the phenyl group in the above-mentioned benzoyl group, phenyl-C₁₋₄ alkyl group or phenyl group may have substituted group(s) selected from the group consisting of lower alkoxy, lower alkyl, halogen, di-lower alkylamino, nitro, and lower alkenedioxy.
in formula IV
R₁₁ is chloro and R₁₂ is diethanolamino, or
R₁₁/R₁₂ are identical and are selected from allyl, halo, diethanolamino, solketalo and a group having the formula -O-R_{z} or -NHR_{Z},
R_{z} is selected from alkyl, hydroxyalkyl, alkoxyalkyl, dialkoxyalkyl and 2-oxo-alkyl wherein the or each alkyl and/or alkoxy moiety has less than six carbon atoms, and
R₁₃/R₁₄ are identical and are selected from piperidino, N-tetrahydroisoquinolyl, and a benzylamino group having the following structural formula: 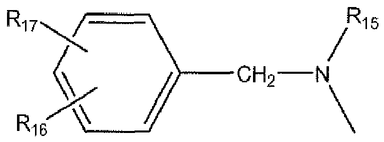
where R₁₅ is H, or an optionally substituted alkyl or benzyl group, and R₁₆ and R₁₇ represent H or optional substituents in the aromatic nucleus selected from halo, alkyl, alkoxy, hydroxy, trifluoromethyl, azido, cyano, nitro, carboxyl, carboxylic ester, amino or a substituted amino NR_{X}R_{Y} where R_{X} and R_{Y} each represent hydrogen or alkyl,
in formula V
R₁' is a C₁₋₆ alkyl, C₂₋₆ alkenyl, C₂₋₆ alkenyl, C₃₋₈-cycloalkyl or a phenyl group, each group being optionally substituted by one or more substituents selected from halogen, OR₈', NR₉' R₁₀', SR₁₁' or C₁₋₆ alkyl (itself optionally substituted by one or more halogen atoms);
R₂' is C₁₋₈ alkyl optionally substituted by one or more substituents selected from halogen, OR₈', NR₉'R₁₀', SR₁₁', C₃₋₈-cycloalkyl, aryl (optionally substituted by one or more alkyl groups and/or halogen atoms), or C₁₋₆-alkyl; or R₂' is a C₃₋₈-cycloalkyl group optionally substituted by one or more substituents selected from halogen, OR₈', NR₉'R₁₀', SR₁₁', C₁₋₆-alkyl or phenyl, the latter two groups being optionally substituted by one or more substituents selected from halogen, NO₂, C(O)R₈', OR₈', SR₁₁', NR₁₂'R₁₃', a fused 5- or 6- membered saturated ring containing one or two oxygen atoms, phenyl or C₁₋₆-alkyl the latter two groups being optionally substituted by OR₈', NR₉'R₁₀' or one or more halogen atoms;
R₃'/R₄' one of them is hydroxy and the other is hydrogen, hydroxy or NR₉'R₁₀';
R' is a group (CR₅'R₆')ₖOR₇' where k is 0 or 1, R₅' and R₆' are independently hydrogen, C₁₋₆ alkyl or phenyl the latter two groups being optionally substituted by halogen, and R₇' is hydrogen, C₁₋₆ alkyl or (CR₅'R₆')ⱼR₁₄' where R₅' and R₆' are as defined above, j is 1 to 3 and R₁₄' is COOH, OR₁₅', NR₁₆'R₁₇' or CONR₁₆'R₁₇'; or R' is a C₁₋₄ alkyl or C₂₋₄ alkenyl group, each or which is substituted by one or more groups selected from =S, =O, =NR₂₀' or OR₂₁' and optionally substituted by one or more groups selected from halogen, C₁₋₄ alkyl or phenyl; SR₂₁', NO₂ or NR₂₂'R₂₃' (where R₂₁', R₂₂' and R₂₃' are independently hydrogen, C₁₋₄ alkyl or phenyl; R₂₀' is OR₂₄' or NR₂₅'R₂₆', where R₂₄' is hydrogen, C₁₋₄ alkyl or phenyl, and R₂₅' and R₂₆' are independently hydrogen, C₁₋₄ alkyl, aryl, C₁₋₆ acyl, arylsulphonyl or arylcarbonyl);
R₈' is hydrogen, C₁₋₆ alkyl optionally substituted by halogen or R₈' is phenyl optionally substituted by one or more substituents selected from halogen, NO₂, C(O)R₆', OR₆', SR₉', NR₁₀'R₁₁';
R₉'/R₁₀'^{/}R₁₁' are independently hydrogen or C₁₋₆ alkyl;
R₁₂'^{/}R₁₃' are independently hydrogen, C₁₋₆ alkyl, acyl, alkyl sulfonyl optionally substituted by halogen, or phenyl sulfonyl optionally substituted by C₁-C₄ alkyl; and
R₁₅'/R₁₆'/R₁₇' are independently hydrogen C₁₋₆ alkyl.
